(19) Europäisches Patentamt
European Patent Office
Office européen des brevets

(11) EP 1 267 170 A1

(12) EUROPEAN PATENT APPLICATION

(43) Date of publication:
**18.12.2002 Bulletin 2002/51**

(51) Int Cl.7: **G01N 33/68**, C07K 1/12

(21) Application number: **01304975.4**

(22) Date of filing: **07.06.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Xzillion GmbH & CO.KG**
**65926 Frankfurt am Main (DE)**

(72) Inventors:
- **Schmidt, Gunter**
  **Houghton, Cambridgeshire PE17 2BQ (GB)**
- **Johnstone, Robert Alexander Walker**
  **Bebington, L63 9LD (GB)**
- **Thompson, Andrew Hugin**
  **Alloway, Ayr KA7 4RH (GB)**
- **Hamon, Christian**
  **65934 Frankfurt am Main (DE)**
- **Neumann, Thomas**
  **65929 Frankfurt am Main (DE)**
- **Joubert, Richard**
  **65931 Frankfurt am Main (DE)**

(74) Representative: **Hill, Christopher Michael et al**
**Page White & Farrer**
**54 Doughty Street**
**London WC1N 2LS (GB)**

(54) **Method for characterising polypeptides**

(57) Provided is a method for characterising a polypeptide or a population of polypeptides, which method comprises the steps of:

(a) contacting a sample comprising one or more polypeptides with a lysine reactive agent to cap ε-amino groups;
(b) optionally reacting the sample of polypeptides with an amine reactive reagent to block α-amino groups;
(c) digesting the sample of polypeptides with a sequence specific cleavage reagent to produce peptide fragments;
(d) optionally deactivating the cleavage reagent;
(e) removing those peptides having uncapped or unblocked amino groups; and
(f) recovering the N-terminal peptides.

EP 1 267 170 A1

## Description

This invention relates to methods of isolating a single terminal peptide from each protein in a population. This invention further relates to the use of the above methods in methods of determining the expression of proteins in a tissue, cell type, or sub-cellular compartment or in analysing large protein complexes. In particular, the present invention is concerned with distinguishing between α-amino groups in the peptides and ε-amino groups in lysine residues, which could otherwise hinder the characterisation process.

Techniques for profiling proteins, that is to say cataloguing the identities and quantities of proteins in a tissue, are not well developed in terms of automation or high throughput. A typical method of profiling a population of proteins is by two-dimensional electrophoresis (R.A. Van Bogelen., E.R. Olson, "Application of two-dimensional protein gels in biotechnology", Biotechnol Annu. Rev., 1, 69-103, 1995). In this method, a protein sample extracted from a biological sample is separated on a narrow gel strip. This first separation usually separates proteins on the basis of their iso-electric point. The entire gel strip is then laid against one edge of a rectangular gel. The separated proteins in the strip are then electrophoretically separated in the second gel on the basis of their size. This technology is slow and very difficult to automate. It is also relatively insensitive in its simplest embodiments. A number of improvements have been made to increase resolution of proteins by 2-D gel electrophoresis and to improve the sensitivity of the system. One approach to improve the sensitivity of 2-D gel electrophoresis and its resolution is to analyse the protein in specific spots on the gel by mass spectrometry (Jungblut P, Thiede B. "Protein identification from 2-D gels by MALDI mass spectrometry." Mass Spectrom. Rev. 16, 145-162, 1997. One example of a mass spectrometry method is in-gel tryptic digestion followed by analysis of the tryptic fragments by mass spectrometry to generate a peptide mass fingerprint. If sequence information is required, tandem mass spectrometry analysis can be performed.

More recently attempts have been made to exploit mass spectrometry to analyse whole proteins that have been fractionated by liquid chromatography or capillary electrophoresis (Dolnik V. "Capillary zone electrophoresis of proteins.", Electrophoresis 18, 2353-2361, 1997). In-line systems exploiting capillary electrophoresis mass spectrometry have been tested. The analysis of whole proteins by mass spectrometry, however, suffers from a number of difficulties. The first difficulty is the analysis of the complex mass spectra resulting from multiple ionisation states accessible by individual proteins. The second major disadvantage is that the mass resolution of mass spectrometers is at present quite poor for high molecular weight species, i.e. for ions that are greater than about 4 kilodaltons (kDa) in mass, so resolving proteins that are close in mass is difficult. A third disadvantage is that further analysis of whole proteins by tandem mass spectrometry is difficult as the fragmentation patterns for whole proteins are extremely complex and difficult to interpret.

As a result of the difficulties of analysing whole proteins, techniques that rely on the analysis of peptides from proteins are preferred. Peptide mass fingerprinting has been used in the analysis of gel separated proteins as described above. However, this process is adequate only for the analysis of individual proteins or very simple mixtures of proteins. A typical protein will give rise to from twenty to thirty peptides after cleavage with trypsin. The pattern of peptide masses is useful for identifying single proteins, but the complexity of the mass spectrum of the trypsin digest of a mixture of proteins rapidly rises in complexity as the number of proteins in the mixture increases. This increases the chance that a peptide mass is assigned incorrectly to a protein, thus limiting the number of proteins that may be analysed simultaneously. As a result new protein characterisation methods are being developed in which specific peptides are isolated from each protein in a mixture.

Nature Biotechnology 17, 994 - 999 (1999) discloses the use of 'isotope encoded affinity tags' for the capture of peptides from proteins, to allow protein expression analysis. In this article, the authors describe the use of a biotin linker, which is reactive to thiols, for the capture peptides with cysteine in them. A sample of protein from one source is reacted with the biotin linker and cleaved with an endopeptidase. The biotinylated cysteine-containing peptides can then be isolated on avidinated beads for subsequent analysis by mass spectrometry. Two samples can be compared quantitatively by labelling one sample with the biotin linker and labelling the second sample with a deuterated form of the biotin linker. Each peptide in the samples is then represented as a pair of peaks in the mass spectrum where the relative peak heights indicate their relative expression levels.

This 'isotope encoding' method has a number of limitations. A first is the reliance on the presence of thiols in a protein - many proteins do not have thiols while others have several. In a variation on this method, linkers may be designed to react with other side chains, such as amines. However, since many proteins contain more than one lysine residue, multiple peptides per protein would generally be isolated in this approach. It is likely that this would not reduce the complexity of the sample sufficiently for analysis by mass spectrometry. A sample that contains too many species is likely to suffer from 'ion suppression', in which certain species ionise preferentially over other species which would normally appear in the mass spectrum in a less complex sample. In general, capturing proteins by their side chains is likely to give either too many peptides per protein or certain proteins will be missed altogether.

The second limitation of this approach is the method used to compare the expression levels of proteins from different samples. Labelling each sample with a different isotope variant of the affinity tag results in an additional peak

in the mass spectrum for each peptide in each sample. This means that if two samples are analysed together there will be twice as many peaks in the spectrum. Similarly, if three samples are analysed together, the spectrum will be three times more complex than for one sample alone. It is clear that this approach will be limited, since the ever increasing numbers of peaks will increase the likelihood that two different peptides will have overlapping peaks in the mass spectrum.

A further limitation, which is reported by the authors of the above paper, is the mobility change caused by the tags. The authors report that peptides labelled with the deuterated biotin tag elute slightly after the same peptide labelled with the undeuterated tag.

Published international patent application WO 98/32876 discloses methods of profiling a population of proteins by isolating a single peptide from one terminus of each protein in the population. In a first aspect the invention comprises the steps of:

1. capturing a population of proteins onto a solid phase support by one terminus of each protein in the population;
2. cleaving the captured proteins with a sequence specific cleavage agent;
3. washing away peptides generated by the cleavage agent not retained on the solid phase support;
4. releasing the terminal peptides retained on the solid phase support; and
5. analysing the released terminal peptides, preferably identifying and quantifying each peptide in the mixture. The analysis is preferably performed by mass spectrometry.

In this application, the C-terminus is discussed as being more preferable as the terminus by which to capture a population of proteins, since the N-terminus is often blocked. In order to capture a population of proteins by the C-terminus, the C-terminal carboxyl group must be distinguished from other reactive groups on a protein and must be reacted specifically with a reagent that can effect immobilisation. In many C-terminal sequencing chemistries the C-terminal carboxyl group is activated to promote formation of an oxazolone group at the C-terminus. During the activation of the C-terminal carboxyl, side chain carboxyls are also activated, but these cannot form an oxazolone group. It has been reported that the C-terminal oxazolone is less reactive to nucleophiles under basic conditions than the activated side-chain carboxyls, offering a method of selectively capping the side chain carboxyl groups (V. L. Boyd et al., Methods in Protein Structure Analysis: 109-118, Plenum Press, Edited M. Z. Atassi and E. Appella, 1995). Other more reactive side chains can be capped prior to the activation of the carboxyls using a variety of conventional reagents. In this way all reactive side chains can be capped and the C-terminus can be specifically labelled.

EP A 0 594 164 and EP B 0 333 587 describe methods of isolating a C-terminal peptide from a protein in a method to allow sequencing of the C-terminal peptide using N-terminal sequencing reagents. In this method the protein of interest is digested with an endoprotease, which cleaves at the C-terminal side of lysine residues. The resultant peptides are reacted with diisothiocyanato (DITC) polystyrene which reacts with all free amino groups. N-terminal amino groups that have reacted with the DITC polystyrene can be cleaved with trifluoroacetic acid (TFA) thus releasing the N-terminus of all peptides. The epsilon-amino group of lysine is not cleaved however and all non-terminal peptide are thus retained on the support and only C-terminal peptides are released. According to this patent the C-terminal peptides are recovered for micro-sequencing.

Anal. Biochem. **132:** 384-388 (1983) and DE A 4344425 (1994) describe methods of isolating an N-terminal peptide from a protein by reacting the protein with a capping reagent which will cap any free amino groups in the protein. The protein is then cleaved, and if trypsin is used cleavage occurs only at arginine residues. Cleavage with trypsin thus exposes $\alpha$-amino groups in the non-N-terminal peptides. In the first disclosure (Anal. Biochem.) the $\alpha$-amino groups are reacted with dinitrofluorobenzene (DNF) which allows the non-N-terminal peptides to be captured by affinity chromatography onto a polystyrene resin while the N-terminal peptides flow through unimpeded. In DE A 4344425, the epsilon amino groups are reacted with an acylating agent prior to cleavage. After cleavage in this method, the $\alpha$-amino groups on the non-N-terminal peptides are reacted with an amine reactive solid support such as diisothiocyanato glass, leaving the N-terminal peptides free in solution.

The main drawback of all of these N-terminal isolation methods is the use of acylating reagents which tend to be unstable in aqueous conditions at the pH needed for lysine modification. As a result, large excesses of reagent need to be used which can lead to side-reactions particularly with histidine residues. The Anal. Biochem. method also requires that the DNF groups be removed from histidine and tyrosine by thiolysis prior to isolating the N terminal peptide, if the N terminal peptide contains these groups. This additional step requires extra effort and may not go to completion. In the Anal. Chem. disclosure the protein and terminal peptides are not analysed by mass spectrometry and so it is not possible to know whether the capping of the lysine epsilon amino groups goes to completion.

It is an aim of this invention to solve the problems associated with the known methods described above. It is thus an aim of this invention to provide improved methods for isolating a single terminal peptide from each protein in a mixture of polypeptides using protein reactive reagents that are stable in water, selective for lysine and that work under mild reaction conditions without degradation of the reagents.

Accordingly, the present invention provides a method for characterising a polypeptide or a population of polypeptides, which method comprises the steps of:

(a) contacting a sample comprising one or more polypeptides with a lysine reactive agent to cap ε-amino groups;
(b) optionally reacting the sample of polypeptides with an amine reactive reagent to block α-amino groups;
(c) digesting the sample of polypeptides with a sequence specific cleavage reagent to produce peptide fragments;
(d) optionally deactivating the cleavage reagent;
(e) removing those peptides having uncapped or unblocked amino groups; and
(f) recovering the N-terminal peptides.

This method allows lower concentrations of the reagents to be used at higher pH. Both of these factors have been found by the inventors to improve the selectivity and completeness of lysine reactions. In the following description, lysine amino groups will be referred to as epsilon amino (ε-amino) groups.

The lysine reactive agent is preferably a hindered Michael reagents. A Michael reagent has a general formula as below:

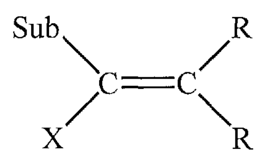

In the above formula, X is an electron withdrawing group that is capable of stabilising a negative charge. The functional group -X is preferably selected from those listed in Table 1 below:

Table 1

| Functional Group | Structure |
|---|---|
| Aldehyde | 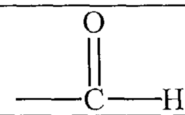 |
| Amide | 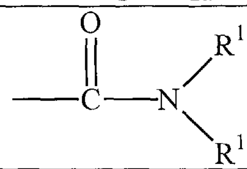 |
| Ester | 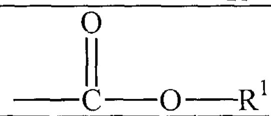 |
| Ketone | 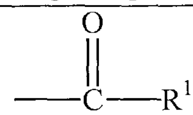 |
| Nitrile | 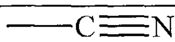 |
| Pyridine ring | 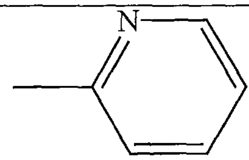 or 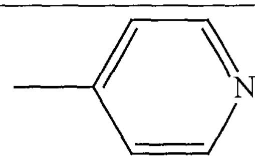 |
| Sulphone | 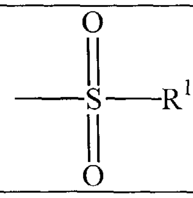 |

Where $R^1$ may be any alkyl or aromatic group but is preferably an electron withdrawing group and more preferably a cyclic or heterocylic aromatic ring or fused ring. Preferably the ring structure is electron withdrawing. More specifically $R^1$ is preferably a small ring or fused ring such as a phenyl, pyridyl, naphthyl or quinolyl ring structure. Preferred ring structures are substituted with appropriate electron withdrawing groups such as halogens like fluorine or nitro groups. Preferred ring structures promote water solubility, such as pyridyl and naphthyl rings. If -X is an amide, then one or both of the $R^1$ groups may be a hydrogen atom. If -X is a nitrile, preferred compounds include crotonitriles such as trifluorocrotonitrile.

To be a 'hindered' Michael reagent according to this invention, at least one of the R groups is not hydrogen and is considered to be a sterically hindering group. At least one R group may comprise an alkyl or aromatic group such as a methyl or phenyl group. More preferably at least one of the R groups is electron-withdrawing and may comprise a halogen atom or a halogenated alkyl group, such as fluoromethyl, difluoromethyl or trifluoromethyl group or a phenyl ring with electron withdrawing substituents such as halogen or nitro groups. Conversely to be an 'unhindered' Michael reagent in the context of this invention, both R groups would be hydrogen.

In some embodiments, the X group may be joined to one of the R groups to form a ring. Preferred compounds of this type include maleimides of the formula:

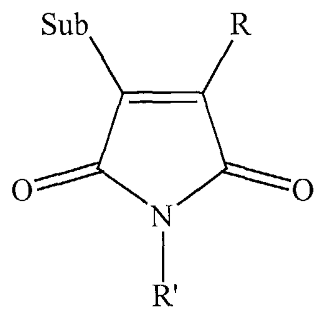

Where R has the same meaning as above and R' is a hydrocarbon group or an electron donating group. Preferably R comprises an alkyl group or aryl group and particularly preferably R comprises a $C_1$-$C_6$ alkyl group, such as a methyl or ethyl group.

The group Sub in the above formulae is not particularly limited, provided that the Michael agent is capable of reacting with an ε-amino group. In preferred embodiments of the invention, Sub comprises a hydrocarbon group such as an alkyl or aryl group or an electron withdrawing group, such as a cyano group (-CN), or a halogen (F, Cl, Br, I) or halogen-containing group. In the most preferred embodiments, Sub comprises a hydrogen, or a $C_1$-$C_6$ alkyl group, such as a methyl or ethyl group. A particularly preferred compound is one in which Sub and R are both H and R' comprises a methyl group or an ethyl group.

In the context of this invention, the term lysine-selective reagent refers to the ability of the reagent to discriminate between the epsilon-amino group of lysine and the alpha-amino groups of all amino acids, and in particular the α-amino group of an N-terminal amino acid residue in a peptide. It is also preferred that the reagents of this invention do not react with other side chain functionalites such as the imidazole ring of histidine and hydroxyl functionalities found in serine, threonine and tyrosine.

The various aspects of this invention will now be discussed in more detail below.

In one embodiment of this invention there is provided a method of isolating a population of naturally blocked and unblocked N-terminal peptides from a sample of polypeptides comprising the steps of:

1. reacting a sample of polypeptides with a lysine-selective hindered Michael reagent so that all available epsilon-amino groups in the polypeptides are capped with the reagent and preferably only one molecule of the alkylating Michael reagent reacts with each epsilon-amine available in the polypeptides;
2. reacting the sample of polypeptides with an amine reactive reagent that will block any naturally unblocked alpha-amino groups (i.e. naturally unblocked N termini);
3. digesting the sample of polypeptides completely with a sequence specific cleavage reagent;
4. contacting the resultant capped peptides with either:

    (a) a solid support or
    (b) a capture reagent,

    that will react with primary amino groups thus capturing free α-amino groups in N-terminal peptides that are not blocked naturally or free α-amino groups that are exposed by the cleavage reagent or any epsilon amino groups that were not blocked in the first reaction step;
5. recovering the blocked and epsilon-amino-capped N-terminal peptides, which should not have a free amine to react with a solid support or capture reagent.

The N-terminal peptides that have been recovered in this and other embodiments of the present invention (described below) are preferably identified using mass spectrometry. For this reason it is preferred that only one Michael agent reacts per ε-amino acid residue. This ensures that only a single peak appears in the mass spectrum for this residue, simplifying the total spectrum and facilitating identification of the N-terminal residues. Employing a hindered Michael agent ensures that a one-to-one reaction with the ε-amino acid residue is promoted. Thus, in the context of the present invention, hindered means sufficiently hindered to promote a one-to-one reaction with the ε-amino group of a lysine residue.

To provide a schematic view of the protocols of the present invention, this first procedure is summarised according to scheme 1:

Scheme 1

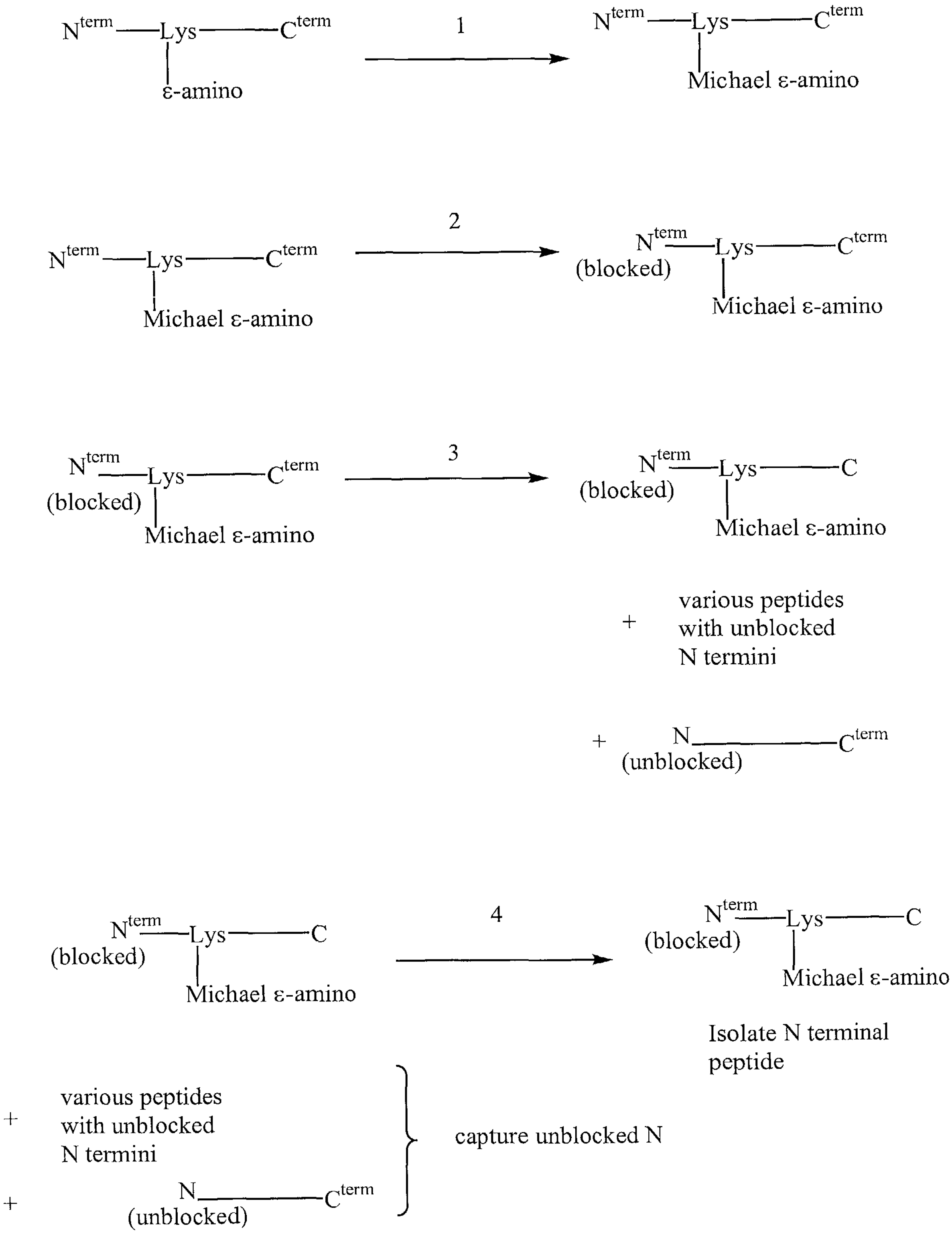


A further aspect of this embodiment of the invention relates only to naturally blocked N-terminal peptides and thus does not make use of the α-amino blocking step 2. This aspect provides a method of isolating a population of naturally blocked N-terminal peptides from a sample of polypeptides comprising the steps of:

1. reacting a sample of polypeptides with a lysine-selective hindered Michael reagent so that all available epsilon-amino groups in the polypeptides are capped with the reagent and preferably only one molecule of the alkylating Michael reagent reacts with each epsilon-amine available in the polypeptides;
2. digesting the sample of polypeptides completely with a sequence specific cleavage reagent;
3. contacting the resultant capped peptides with either

(a) a solid support or
(b) a capture reagent

that will react with primary amino groups thus capturing free α-amino groups in N-terminal peptides that are not blocked naturally or free α-amino groups that are exposed by the cleavage reagent, or any epsilon amino groups that were not blocked in the step 1; and
4. recovering the blocked and epsilon-amino capped N-terminal peptides, which should not have a free amine to react with a solid support or capture reagent.

In a still further aspect of this embodiment, this invention provides a method of isolating a population of unblocked N-terminal peptides from a sample of polypeptides comprising the steps of:

1. reacting the sample of polypeptides with a lysine-selective hindered Michael reagent so that all available epsilon-amino groups in the polypeptides are capped with the reagent and preferably only one molecule of the alkylating reagent reacts with each epsilon-amine available in the polypeptides;
2. contacting the resultant capped polypeptides with either a primary amine reactive capture reagent or a solid support to react with any unblocked free alpha-amino groups at the N-termini of any of the polypeptides;
3. digesting the sample of polypeptides completely with a sequence specific cleavage agent; and
4. recovering the unblocked and epsilon-amino capped N-terminal peptides that have been derivitised with the capture reagent.

In a still further embodiment, this invention provides a method of determining the 'expression profile' of at least one mixture of polypeptides, i.e. a method to identify and preferably also to quantify each polypeptide in the mixture. This method comprises the following steps:

1. isolating terminal peptides according to any one of the previous embodiments of this invention from at least one mixture of polypeptides;
2. optionally labelling the free alpha amino group of the recovered C-terminal peptides from each sample with a different mass marker;
3. optionally separating the C-terminal peptides electrophoretically or chromatographi cally;
4. detecting the peptides by mass spectrometry.

In a yet further embodiment, this invention provides a lysine selective protein labelling reagent that comprises a thiol and amino reactive hindered alkenyl sulphone compounds with the formula:

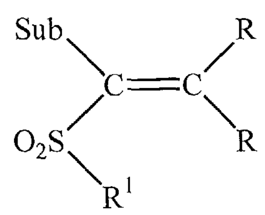

Where $R^1$ may be any alkyl or aromatic group but is preferably an electron withdrawing group and more preferably a cyclic or heterocylic aromatic ring or fused ring. Preferably the ring structure is electron withdrawing. More specifically $R^1$ is preferably a small ring or fused ring such as a phenyl, pyridyl, naphthyl or quinolyl ring structure. Preferred ring structures are substituted with appropriate electron withdrawing groups such as halogens like fluorine or nitro groups. Preferred ring structures promote water solubility such as pyridyl and naphthyl rings.
At least one of the R groups is not hydrogen and is considered to be a sterically hindering group. At least one R group may comprise an alkyl or aromatic group such as a methyl or phenyl group. More preferably at least one of

the R groups is electron-withdrawing and may comprise a halogen atom or a halogenated alkyl group, such as fluoromethyl, difluoromethyl or trifluoromethyl group or a phenyl ring with electron withdrawing substituents such as halogen or nitro groups. Conversely to be an 'unhindered' Michael reagent in the context of this invention, both R groups would be hydrogen.

The group Sub in the above formula is not particularly limited, provided that the Michael agent is capable of reacting with an ε-amino group. In preferred embodiments of the invention, Sub comprises a hydrocarbon group such as an alkyl or aryl group or an electron withdrawing group, such as a cyano group (-CN), or a halogen (F, Cl, Br, I) or halogen-containing group. In the most preferred embodiments, Sub comprises a hydrogen, or a $C_1$-$C_6$ alkyl group, such as a methyl or ethyl group. A particularly preferred compound is one in which Sub and R are both H and R' comprises a methyl group or an ethyl group.

The invention will now be described in more detail by way of example only, with reference to the following Figures:

Figure 1 shows a selection of preferred hindered alkenyl sulphone reagents for use with this invention - synthetic procedures for the production of some of these reagents are described in the examples;

Figure 2a shows the first page of an illustration of the first embodiment of this invention using α-MSH and β-MSH as examples;

Figure 2b shows the second page of an illustration of the first embodiment of this invention using α-MSH and β-MSH as examples;

Figure 2c shows the third page of an illustration of the first embodiment of this invention using α-MSH and β-MSH as examples;

Figure 3a shows the first page of an illustration of the second embodiment of this invention using α-MSH and β-MSH as examples;

Figure 3b shows the second page of an illustration of the second embodiment of this invention using α-MSH and β-MSH as examples;

Figure 4a shows the first page of an illustration of the third embodiment of this invention using α-MSH and β-MSH as examples;

Figure 4b shows the second page of an illustration of the third embodiment of this invention using α-MSH and β-MSH as examples;

Figure 4c shows the third page of an illustration of the third embodiment of this invention using α-MSH and β-MSH as examples;

Figure 5 shows the mass spectrum of an example of a protocol for labelling both the thiols and epsilon amino groups of a peptide - in this example the thiols are labelled with a different tag from the epsilon amino groups;

Figure 6 shows the mass spectrum of an example of a protocol for labelling both the thiols and epsilon amino groups of a peptide with the same label;

Figure 7 shows the mass spectrum of an example of a protocol for labelling both the thiols and epsilon amino groups of a mixture of peptides - in this example the thiols are labelled with the same tag as the epsilon amino groups;

Figure 8 shows the mass spectrum of an example of a protocol for labelling the alpha-amino groups of a mixture of peptides where both the thiols and epsilon-amino groups of the peptides have already been blocked with the same mass tag;

Figure 9 shows the mass spectrum of an example of the first aspect of this invention in which N-terminal peptides were isolated from a small mixture of larger peptides after enzymatic cleavage with trypsin - this figure shows region of a MALDI TOF spectrum with the expected peaks for the N-terminal peptides of α-MSH, β-MSH and ACTH (1-24);

Figure 10 is from the same experiment as Figure 9 showing the region of the spectrum with the expected peaks for the N-terminal peptides of Calcitonin S, Calcitonin H - the expected peaks and some extra labelling peaks are found;

Figure 11 is from the same experiment as Figure 10 showing the low mass region of the spectrum where any contaminating C-terminal peptides would be found if they were present;

Figure 12 shows the mass spectrum of an example of the first aspect of this invention in which the N-terminal peptide was isolated from human Calcitonin after chemical cleavage of the peptide with cyanogen bromide;

Figures 2, 3 and 4 will now be described in more detail. Figures 2a to 2c illustrate one embodiment of this invention, which provides a method of isolating a population of naturally blocked and unblocked N-terminal peptides from a sample of polypeptides. Figure 2a illustrates the first step of this process in which two peptides are reacted with a hindered alkenyl sulphone. Two peptides, rather than a complex mixture, are shown, alpha-melanocyte stimulating hormone (α-MSH) and beta-melanocyte stimulating hormone (β-MSH), for ease of illustration. These peptides represent the pools of blocked and unblocked polypeptides respectively that would be present in a natural sample. Pyridyl propenyl sulphone is a preferred lysine-selective hindered Michael reagent according to this invention. This reagent reacts highly selectively and almost completely with lysine epsilon-amino groups in preference to unblocked alpha-amino groups.

Figure 2b illustrates the second step of this embodiment of the invention in which the blocked and unblocked peptides are reacted with acetic acid N-hydroxysuccinimide ester. This reagent does not show significant selectivity for either alpha-amino groups or epsilon-amino groups, but since the epsilon-amino groups are already blocked the reagent reacts with any naturally unblocked alpha-amino groups present in the sample. In the figure the N-terminus of β-MSH is unblocked an is capped by this reaction. Figure 2b also illustrates the third step of this embodiment of the invention in which the polypeptides, which now have all free amines capped, are cleaved with a sequence specific cleavage reagent. In the figure this step is performed either with trypsin, which will now only cut the capped peptides at arginine, or with Arg-C, which only cuts at arginine. The cleavage reaction generates new free alpha-amino groups in the C-terminal product peptides of each cut. This means that no new amine is exposed in the N-terminal peptides, but all other peptides will now have a free amino group.

Figure 2c illustrates the fourth step of this embodiment of the invention in which the amino groups exposed by the previous cleavage step are reacted with a capture reagent. In the figure this reagent is biotin N-hydroxysuccinimide ester, a well known affinity capture reagent that will react with primary amino groups. Since all non-N-terminal peptides have a free primary amino group, these peptides will react with the biotin reagent. The N-terminal peptides will not be biotinylated. Figure 2c also illustrates the final step in this embodiment of the invention in which the blocked N-terminal peptides are separated from biotinylated non-N-terminal peptides by passing the products of the biotinylation reaction through an avidin affinity column. The biotinylated non-N-terminal peptides will adhere to the column while the N-terminal peptides will elute from the column and can be recovered for analysis.

Figures 3a and 3b illustrate a second embodiment of this invention, which provides a method of isolating a population of naturally blocked peptides from a sample of polypeptides comprising a mixture of blocked and unblocked species. Figure 3a illustrates the first step of this process in which two peptides are reacted with a hindered alkenyl sulphone. Again only two peptides, rather than a complex mixture, are shown, α-MSH and β-MSH, for ease of illustration. These peptides represent the pools of blocked and unblocked polypeptides respectively that would be present in a natural sample. The hindered alkenyl sulphone is a preferred lysine-selective hindered Michael reagent according to this invention. This reagent reacts highly selectively and almost completely with lysine epsilon-amino groups in preference to unblocked alpha-amino groups.

Figure 3b illustrates the second step of this embodiment of the invention in which the polypeptides, which now have all lysine amino groups capped, are cleaved with a sequence specific cleavage reagent. In the figure this step is performed either with trypsin, which will now only cut the capped peptides at arginine, or with Arg-C, which only cuts at arginine. The cleavage reaction generates new free alpha-amino groups in the C-terminal product peptides of each cut. This means that no new amine is exposed in the naturally blocked N-terminal peptides, i.e. the N-terminal peptide of α-MSH, but all other peptides will now have a free amino group. Any naturally unblocked N-terminal peptides will also have a free alpha-amino group, i.e. the N terminal peptide of β-MSH. Figure 3b also illustrates the third step of this embodiment of the invention in which all free alpha-amino groups are reacted with a capture reagent. In the figure this reagent is biotin N-hydroxysuccinimide ester, a well known affinity capture reagent that will react with primary amino groups. Since naturally unblocked peptide and all non-N-terminal peptides have a free primary amino group, these peptides will react with the biotin reagent. The naturally blocked N-terminal peptide of α-MSH will not be biotinylated. Figure 3b also illustrates the final step in this embodiment of the invention in which the naturally blocked N-terminal peptides are separated from biotinylated non-N-terminal peptides and the biotinylated N-terminal peptides,

which were naturally unblocked, by passing the products of the biotinylation reaction through an avidin affinity column. The biotinylated non-N-terminal peptides and the biotinylated N-terminal peptides, which were naturally unblocked, will adhere to the column while the naturally blocked N-terminal peptides, i.e. the N-terminal peptide of α-MSH, will elute from the column and can be recovered for analysis.

Figures 4a to 4c illustrate a third embodiment of this invention, which provides a method of isolating a population of naturally unblocked peptides from a sample of polypeptides comprising a mixture of blocked and unblocked species. Figure 4a illustrates the first step of this process in which two peptides are reacted with a hindered alkenyl sulphone. Again only two peptides, rather than a complex mixture, are shown, α-MSH and β-MSH, for ease of illustration. These peptides represent the pools of blocked and unblocked polypeptides respectively that would be present in a natural sample. The hindered alkenyl sulphone is a preferred lysine-selective hindered Michael reagent according to this invention. This reagent reacts highly selectively and almost completely with lysine epsilon-amino groups in preference to unblocked alpha-amino groups.

Figure 4b illustrates the second step of this embodiment of the invention in which the polypeptides, which now have all lysine amino groups capped, are reacted with a capture reagent. In the figure this reagent is biotin N-hydroxysuccinimide ester, a well known affinity capture reagent that will react with primary amino groups. Since only naturally unblocked peptides, i.e. β-MSH, will have a free primary amino group, these peptides will react with the biotin reagent. The naturally blocked N-terminal peptides, i.e. α-MSH will not be biotinylated. Figure 4b also illustrates the third step of this embodiment of the invention in which the capped and biotinylated peptides are cleaved with a sequence specific cleavage reagent. In the figure this step is performed either with trypsin, which will now only cut the capped peptides at arginine, or with Arg-C, which only cuts at arginine. The cleavage reaction generates new free alpha-amino groups in the C-terminal product peptides of each cut. This means that no new amine is exposed in the N-terminal peptides, but all other peptides will now have a free amino group.

Figure 4c illustrates the final step in this embodiment of the invention in which the biotinylated peptides, which were naturally unblocked N-terminal peptides, are separated from non-N-terminal peptides and the naturally blocked N-terminal peptides by passing the products of the biotinylation reaction through an avidin affinity column. The biotinylated N-terminal peptides, which were naturally unblocked, i.e. the N-terminal peptide of β-MSH, will adhere to the column while the naturally blocked N-terminal peptides and non-N-terminal peptides will elute from the column. The N-terminal peptides which were naturally unblocked can be recovered for analysis by acidification of the avidin column or denaturation or by addition of excess biotin. Alternatively a cleavable form of biotin can be used, such as EZ-Link® Sulfo-NHS-SS-Biotin (Pierce & Warriner UK Ltd, Chester, UK) which is a biotin N-hydroxysuccinimide ester compound with a disulphide linker that is cleavable with reducing agents. This reagent is advantageous as the released peptide has a free thiol from the cleavage of the disulphide linkage. This free thiol provides a reactive group for the introduction of a label into the released peptides if desired. The recovered naturally unblocked peptides can then be analysed further. The eluent of naturally blocked and non-N-terminal peptides can also be analysed further if desired. This pool of peptides can be biotinylated again. The blocked N-terminal peptides cannot react with biotin and so after passing the products of the biotinylation reaction through an avidinated column only the blocked N-terminal peptides will elute.

The lysine reactive (lysine selective) reagents used in the methods of the present invention will now be described in more detail.

Many amine selective protein reactive reagents are known in the art. These reagents will all have some degree of discrimination in favour of reaction with lysine at high pH, but not many show sufficient discrimination to allow lysine to be labelled almost exclusively. A number of lysine-selective reagents have been described in the prior art and these are all appropriate for use with this invention, particularly cyclic anhydrides. Pyromellitic dianhydride and *o*-sulphobenzoic acid anhydride are reported to be lysine selective acylating reagents (Bagree et al., FEBS Lett. 120 (2):275-277, 1980). Similarly Phthalic anhydride, whose structure and reactivity is similar to pyromellitic anhydride would be expected to be lysine selective. Phthalic anhydride is reported to have few side-reactions with other amino acids (Palacian E. *et al.*, Mol Cell Biochem. 97 (2): 101-111, 1990). However, many widely used reagents that react with lysine are not stable at high pH, particularly active esters such as carboxylic acid anhydrides, N-hydroxysuccinimide esters and pentafluorophenyl esters. These reagents must be used in large excess exacerbating the lack of selectivity of the reaction as a result of the excess.

Michael reagents have a number of properties that make them attractive for protein reactions and have been used quite widely for this purpose (Friedman M. & Wall J.S., J Org Chem. 31, 2888 - 2894, 'Additive Linear Free-Energy Relationships in Reaction Kinetics of Amino Groups with alpha-,beta-Unsaturated Compounds.' 1966; Morpurgo M. & Veronese F.M. & Kachensky D. & Harris J.M., Bioconjug. Chem. 7(3): 363-368, 'Preparation of characterization of poly (ethylene glycol) vinyl sulfone.' 1996; Friedman M. & Finley J.W., Int. J. Pept. Protein Res. **7**(6): 481 - 486, 'Reactions of proteins with ethyl vinyl sulfone.' 1975; Masri M.S. & Friedman M., J Protein Chem. 7(1): 49-54, 'Protein reactions with methyl and ethyl vinyl sulfones' 1988; Graham L. & Mechanic G. L., Anal. Biochem. 153(2): 354-358, '[14C]acrylonitrile: preparation via a stable tosylate intermediate and quantitative reaction with amine residues in collagen.' 1986; Esterbauer H. & Zollner H. & Scholz N., Z Naturforsch [C] 30(4): 466-473, 'Reaction of glutathione with conjugated

carbonyls.' 1975).

There is a number of these reagents that are relatively stable in aqueous solution and the structures of these compounds can be varied extensively to achieve different degrees of reactivity and selectivity. Other reagents used for protein labelling are often not very stable in water and are less easily modified. In particular, reactions with amino-groups in proteins are often done with active esters, which are quite susceptible to hydrolysis. Reagents based on sulphones may be more convenient and effective for labelling amino-groups than the more widely used active esters. Michael reagents that have been used with proteins include compounds such as acrylonitrile, acrylamide, vinyl pyridine, methylvinyl sulphone and methylvinyl ketone. The reactions of these compounds have been compared (Friedman M. & Wall J.S from above) and linear relationships between the reaction kinetics of these structurally similar compounds are observed. These linear relationships indicate that the reactions of this class of compounds take place by the same mechanism although their rates of reaction differ. The authors found that the sulphone and ketone compounds were by far the most reactive reagents. The vinyl compounds, i.e. acrylonitrile, acrylamide, vinyl pyridine, methylvinyl sulphone and methylvinyl ketone have broadly the same relative rates of reaction with different substrates but differ from each other in their overall rates of reaction. These linear relationships make it reasonable to assume that the reactions of this class of compounds take place by the same mechanism and that changes to substituents in this class of compounds, particularly at the beta position of the reactive double bond, will produce similar changes in behaviour in the whole class of compounds. For example, it would be expected that the change in relative reaction rates of crotononitrile with a series of substrates when compared with acrylonitrile would be essentially the same as the change in relative reaction rates of methyl propenyl sulphone with a series of substrates when compared with methyl vinyl sulphone. This means that the properties of methyl propenyl sulphone will be essentially the same as crotononitrile except that the rate of reaction of the sulphone will be faster.

The choice of a Michael reagent for the purposes of this invention is dependent on a number of criteria, included rates of reaction, chances of side-reactions apart from the Michael addition and ease of synthesis of different variants of the compound. Vinyl ketones can, for example, undergo other reactions besides Michael addition, particularly nucleophilic attack of the ketone after Michael addition has taken place. The ketone functionality can undergo this further reaction with a variety of nucleophiles, including the usual biological nucleophiles. Similarly, nitrile compounds can undergo hydrolysis of the nitrile functionality to the carboxylic acid, although typically this reaction will not occur under the conditions used in most biological assays. Alkenyl sulphones do not undergo reactions other than the Michael addition under the conditions used in typical biological assays. Alkenyl sulphones generally react rapidly with biological nucleophiles and there is an extensive literature on the synthesis of different forms of alkenyl sulphone. For these reasons alkenyl sulphones are preferred Michael Reagents for use in the biological assays of this invention. Maleimide compounds such as N-ethylmaleimide also react rapidly with proteins by Michael addition and are reasonably stable under the conditions used for labelling proteins, although alkaline hydrolysis is observed when these reagents are polymer bound. Thus maleimide compounds are also preferred Michael Reagents for use in the biological assays of this invention. In most circumstances nitrile reagents are also preferred reagents although a nitrile reagent will tend to react more slowly than corresponding sulphones. Similarly acrylamides react still more slowly. These preferences do not mean that the other Michael reagents available are unsuitable for this invention, but for most purposes rapid reaction of the reagents is preferred. Under appropriate conditions almost any of the Michael reagents could be used in the methods of this invention.

A preferred class of lysine-selective reagents for use in this invention are hindered alkenyl sulphones as provided by one embodiment of this invention. Combinations of these reagents under appropriate mild conditions can allow a high degree of discrimination between alpha-amino groups and lysine epsilon-amino groups in amine-labelling reactions. Vinyl sulphones are known to react readily with primary amines giving a di-alkylated product. The inventors have shown that these reagents will react more rapidly with epsilon-amino groups at high pHs (>9.0) than with alpha-amino groups, but the discrimination of these unhindered sulphones, whilst adequate, is not especially marked. More hindered alkenyl sulphones such as propenyl sulphones and butenyl sulphones show a greatly enhanced discrimination in favour of epsilon-amino groups when compared with the vinyl sulphones, and are therefore preferred. In addition, these hindered reagents produce the mono-alkylated product almost exclusively. Moreover, lysine epsilon-amino groups that have been mono-alkylated with some of the more hindered sulphones are resistant to further reaction with other amine reactive reagents. This is an important feature of the preferred reagents of this invention as in most of the aspects of this invention alpha-amino groups are reacted with an amino-reactive capture reagent, such as NHS-biotin, after the epsilon amino-groups have been blocked with the reagents of this invention.

This discrimination by hindered sulphones means that epsilon-amino groups can be selectively labelled in preference to alpha-amino groups under mild aqueous conditions with convenient, stable, water-soluble reagents. If a lysine selective capture reagent is required, the hindered alkenyl sulphone functional groups of this invention can be linked to a solid support. Alternatively an affinity capture reagent can be generated by linking the hindered alkenyl sulphone functional groups of this invention to biotin or digoxigenin, for example.

The phenyl and pyridyl sulphone compounds depicted in Figure 1 are particularly preferred for use in the

present invention. The pyridyl derivatives are especially useful due to their solubility characteristics (water solubility is preferred). The nitrogen in the pyridine ring may be in the ortho, meta or para position relative to the sulphone group, but the meta (3-position) is preferred. One of the R groups attached to the carbon double bond should not be hydrogen, as explained above, and preferred compounds are those where the R group is methyl or trifluoromethyl. The second R group may be hydrogen, but is also preferably methyl or trifluoromethyl.

Numerous methods of synthesising hindered alkenyl sulphones are known in the art. For general reviews of synthetic methods that have been used for the synthesis of alpha-,beta-unsaturated sulphones see Simpkins N., Tetrahedron 46, 6951-6984, 'The chemistry of vinyl sulphones', 1990; and Fuchs P. L. and Braish T. F., Chem. Rev. 86, 903-917, 'Multiply Convergent Synthesis via Conjugate-Addition Reactions to Cycloalkenyl Sulfones', 1986. Preferred hindered alkenyl sulphone compounds of this invention have the formula:

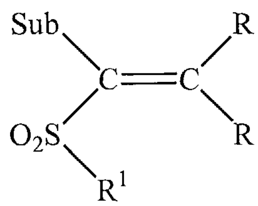

Where $R^1$ is a cyclic or heterocylic aromatic ring or fused ring. Preferably the ring structure is electron withdrawing. More specifically $R^1$ is preferably a small ring or fused ring such as a phenyl, pyridyl, naphthyl or quinolyl ring structure. To make the ring structure electron withdrawing, the ring could be substituted with appropriate electron withdrawing groups such as halogens like fluorine or nitro groups. Pyridyl and naphthyl structures will tend to be more water soluble.

At least one of the R groups is not hydrogen and is considered to be a sterically hindering group. At least one R group may comprise an alkyl or aromatic group such as a methyl or phenyl group. More preferably at least one of the R groups is electron-withdrawing and may comprise a halogen atom or a halogenated alkyl group, such as fluoromethyl, difluoromethyl or trifluoromethyl group or a phenyl ring with electron withdrawing substituents such as halogen or nitro groups. Conversely to be an 'unhindered' Michael reagent in the context of this invention, both R groups would be hydrogen.

The group Sub in the above formula is not particularly limited, provided that the Michael agent is capable of reacting with an ε-amino group. In preferred embodiments of the invention, Sub comprises a hydrocarbon group such as an alkyl or aryl group or an electron withdrawing group, such as a cyano group (-CN), or a halogen (F, Cl, Br, I) or halogen-containing group. In the most preferred embodiments, Sub comprises a hydrogen, or a $C_1$-$C_6$ alkyl group, such as a methyl or ethyl group. A particularly preferred compound is one in which Sub and R are both H and R' comprises a methyl group or an ethyl group.

Various entry points into the synthesis of alkenyl sulphones may be contemplated to produce compounds that are appropriately substituted for use with this invention. Aldol condensation-type reactions can be used. Methyl phenyl sulphone can be reacted with a variety of ketones and aldehydes to give hindered alkenyl sulphones (see Figure 1 and the reviews above). Appropriate ketones include acetone and hexafluoroacetone. Aldehydes include benzaldehyde, fluorobenzaldehyde, difluorobenzaldehyde, trifluoromethylbenzaldehyde and nitrobenzaldehyde. 4-(methylsulfonyl)benzoic acid provides a starting point for the synthesis of a hindered sulphone that can be linked to a solid support or to an affinity capture reagent through the benzoic acid. Amino-derivitised polystyrene is available from various sources including Sigma-Aldrich, UK. Carbodiimide coupling of the functionalised benzoic acid to generate an amide linkage to the solid support would be sufficient to generate a solid support derivitised with the appropriate alkenyl sulphone. Various forms of amino-functionalised biotin are available from Pierce Chemical Company, IL, USA, which would allow a biotin compound derivitised with a variety of alkenyl sulphones to be synthesised.

Synthetic routes for the production of phenyl-1-propenyl, pyridine-1-propenyl, phenyl-1-isobutenyl and pyridine-1-isobutenyl sulphones are described in the Examples below. A synthetic route for the production of 1,1,1-trifluoro-3-phenylsulphonylpropene is disclosed by Tsuge H. *et al.* in J. Chem. Soc. Perkin Trans. **1**:2761 -2766, 1995. This reagent is also available from Aldrich (Sigma-Aldrich, Dorset, UK).

A second preferred class of reagents for use in this invention are maleimide compounds. Combinations of these reagents under appropriate mild conditions can allow a high degree of discrimination between alpha-amino groups and lysine epsilon-amino groups in amine-labelling reactions. Maleimide compounds are known to react readily with primary amines giving a mono-alkylated product. The inventors have shown that a solid support derivitised with maleimide (maleimidobutyramidopolystyrene, Fluka) will react more rapidly with epsilon-amino groups under basic

conditions than with alpha-amino groups. This reagent is not stable in aqueous conditions, however, and reactions of peptides with this support must be carried out in anhydrous aprotic organic solvents.

Some of the less hindered Michael reagents, such as N-ethylmaleimide (NEM) and the propenyl sulphones will react quite readily with the alpha-amino group of proline. This will not be a problem in most aspects of this invention as proline is not common and most endoproteases do not cleave at proline linkages anyway. Trypsin will not cleave at lysine-proline or arginine-proline linkages and is useable in the first and second embodiments of this invention to avoid the production of free proline alpha-amino groups. An N-terminal proline will only be a possible problem for the third embodiment of this invention where unblocked N-terminal peptides are isolated and the isolation relies on discrimination between N-terminal alpha amino groups and epsilon amino groups in the uncleaved protein. Improved proline lysine discrimination is, however, found in the more hindered alkenyl sulphones such as the isobutenyl sulphones, the trifluoropropenyl sulphones and the hexafluoroisobutenyl sulphones, so these reagents should be used if discrimination against proline is required. Solid-support bound maleimide also discriminates effectively against proline.

In the first aspect of the initial embodiment of this invention, which describes a method to isolate all N-terminal peptides from a population of polypeptides, the discrimination of the hindered sulphones is used to protect epsilon-amino groups. This reaction is followed by blocking any naturally unblocked alpha-amino groups with a less selective amine-reactive reagent. Preferred reagents in these circumstances are active esters. The inventors have observed that epsilon-amino groups that have been blocked with hindered reagents are not reactive to active esters and unhindered alkylating reagents despite the amino group still being present. After these two steps substantially all primary amino groups in a polypeptide or mixture of polypeptides should be blocked. The polypeptide can then be cleaved with a sequence specific cleavage reagent, which can be enzymatic such as trypsin or can be chemical such as cyanogen bromide. The cleavage of the mixture of polypeptides with the sequence specific cleavage reagent will expose new alpha-amino groups in all but the N-terminal peptides. These alpha-amino groups can be reacted with a primary-amine reactive solid support or a primary-amine reactive capture reagent. Any primary amino-groups that did not react in earlier steps, e.g. epsilon amino groups will have a second chance to react and will be removed in this capture step, which is advantageous. Again since the epsilon amino groups are blocked with a hindered reagent they will not react with either of these reagents. A variety of primary amine reactive functionalities are known and could be used with this invention to capture peptides with free primary amino groups, although capture reagents that use active esters, such as N-hydroxysuccinimide esters, or unhindered alkylating functionalities, such as vinyl sulphones, may be used. N-hydroxysuccinimide biotin is commercially available (from Pierce UK Ltd, Chester, UK or Sigma-Aldrich, Poole, Dorset, UK) and is widely used, as it has few known side reactions. This capture step will therefore leave all N-terminal peptides free in solution. These may then be labelled further and may be analysed by any appropriate technique, particularly mass spectrometry.

In the second aspect of this embodiment of the invention, which describes a method to isolate the subset of polypeptides that are naturally blocked, the discrimination of the hindered sulphones is used to protect epsilon-amino groups prior to cleavage with a sequence specific cleavage reagent. This leaves any N-terminal unblocked alpha-amino groups free. Only naturally blocked N-terminal peptides will have a blocked alpha-amino group. Cleavage of the mixture of polypeptides with the sequence specific cleavage reagent will expose new alpha-amino groups in all but the naturally blocked N-terminal peptides. This means that a primary-amine reactive solid support or a primary-amine reactive capture reagent such as N-hydroxysuccinimide biotin (available from Pierce UK Ltd, Chester, UK or Sigma-Aldrich, Poole, Dorset, UK) can be used to capture the alpha-amino containing peptides onto a solid support either directly through a covalent bond or via an affinity capture step if an affinity capture reagent is used. Again since the epsilon amino groups are blocked with a hindered reagent they will not react with either of these reagents. This will leave naturally blocked N-terminal peptides free in solution.

In the third aspect of this embodiment, which describes a method to isolate the subset of polypeptides that are naturally unblocked, the discrimination of the hindered sulphones is used to protect epsilon-amino groups. After this treatment free alpha-amino groups on any proteins that are unblocked at the amino terminus can be biotinylated with a primary-amine reactive capture reagent such as N-hydroxysuccinimide biotin (available from Pierce UK Ltd, Chester, UK or Sigma-Aldrich, Poole, Dorset, UK). The proteins are then cleaved and the alpha-amino terminal peptides can be isolated on an avidin column.

In some embodiments of the present invention, the N-terminal peptides are captured on a solid phase. This can be achieved, for example, by reacting the $\alpha$-amino group of the N-terminal amino acid with a biotinylated agent. This biotinylated agent can be captured on an avidinated solid phase, whilst the remaining species in the mixture are washed away. In a preferred embodiment, labelled biotin agents are employed. Differently labelled agents are reacted with different sample and then the samples are pooled and analysed together. The label identifies the sample that the N-terminal residue came from. It is particularly preferred that the method of analysis is mass spectrometry and the type of labelling is isotopic labelling. Thus, in some embodiments, biotin agents are employed with differing levels of deuteration to allow simultaneous analysis of a plurality of samples.

A further embodiment of this invention, provides a method of determining the 'expression profile' of a mixture

of polypeptides, i.e. a method to identify and preferably also to quantify each polypeptide in the mixture. These methods involve isolating peptides according to the first three aspects of the invention, optionally labelling the peptides with a mass marker and analysing the peptides by mass spectrometry. Preferred labels for use with this invention are disclosed in PCT/GB01/01122, which discloses organic molecule mass markers that are analysed by selected reaction monitoring. This application discloses two component mass markers connected by a collision cleavable group. Sets of tags are synthesised where the sum of the masses of the two components produces markers with the same overall mass. The mass markers may be analysed after cleavage from their analyte or may be detected while attached to the analyte. In this invention the mass markers are detected while attached to the peptide that they are identifying. Selection of the mass of the mass marker with its associated peptide by the first mass analyser of a tandem instrument allows the marked peptides to be abstracted from the background. Collision of the markers in the second stage of the instrument separates the two components of the tag from each other. Only one of these components is detected in the third mass analyser. This allows confirmation that the peak selected in the first analyser is a mass marked peptide. The whole process greatly enhances the signal to noise ratio of the analysis and improves sensitivity. This mass marker design also compresses the mass range over which an array of mass markers is spread. Moreover, it allows the design of markers, which are chemically identical, have the same mass but which are still resolvable by mass spectrometry. This is essential for analytical techniques such as Liquid Chromatography Mass Spectrometry (LC-MS) where the effect of different markers on the mobility of different samples of peptides must be minimised so that corresponding peptides from each sample elute together into the mass spectrometer, allowing the ratios of the corresponding peptides to be determined. These markers are thus most preferred for the purposes of this invention because of the use of high selectivity detection and the closely related structures of these markers. Other markers may also be applicable, though.

The reagents of this invention are reactive with free thiols. To prevent interference in the methods of this invention by free thiols and to avoid problems associated with disulphide bridges in polypeptides, it is preferred that the disulphide bridges are reduced to free thiols and that the thiol moieties are capped prior to application of the methods of this invention. Since thiols are very much more reactive than the other side-chains in a protein this step can be achieved highly selectively.

Various reducing agents have been used for disulphide bond reduction. The choice of reagent may be determined on the basis of cost, or efficiency of reaction and compatibility with the reagents used for capping the thiols (for a review on these reagents and their use see Jocelyn P.C., Methods Enzymol. 143, 246-256, 'Chemical reduction of disulfides.' 1987).

Typical capping reagents include N-ethylmaleimide, iodoacetamide, vinylpyridine, 4-nitrostyrene, methyl vinyl sulphone or ethyl vinyl sulphone (see for example Krull L. H. & Gibbs D. E. & Friedman M., Anal. Biochem. 40(1): 80-85, '2-Vinylquinoline, a reagent to determine protein sulfhydryl groups spectrophotometrically.' 1971; Masri M. S. & Windle J. J. & Friedman M., Biochem Biophys. Res. Commun. 47(6): 1408-1413, 'p-Nitrostyrene: new alkylating agent for sulfhydryl groups in reduced soluble proteins and keratins.' 1972; Friedman M. & Zahnley J.C. & Wagner J. R., Anal. Biochem. 106(1): 27-34, 'Estimation of the disulfide content of trypsin inhibitors as S-beta-(2-pyridylethyl)-L-cysteine.' 1980).

Typical reducing agents include mercaptoethanol, dithiothreitol (DTT), sodium borohydride and phosphines such as tributylphosphine (see Ruegg U. T. & Rudinger J., Methods Enzymol. 47, 111-116, 'Reductive cleavage of cysteine disulfides with tributylphosphine.', 1977) and tris(carboxyethyl)phosphine (Burns J.A. *et al.*, J. Org. Chem. 56, 2648-2650, 'Selective reduction of disulfides by tris(2-carboxyethyl)phosphine.', 1991). Mercaptoethanol and DTT may be less preferred for use with thiol reactive capping reagents as these compounds contain thiols themselves.

It is worth noting that the reduction and thiol blocking (of cysteine groups) may take place simultaneously with the epsilon-amino labelling step of the second aspect of this invention. Phosphine based reducing reagents are compatible with vinyl sulphone reagents (Masri M. S. & Friedman M., J. Protein Chem. 7(1), 49-54, 'Protein reactions with methyl and ethyl vinyl sulfones.' 1988). Thus, the thiol groups may be blocked with the same reagents as the epsilon-amino groups. However, thiol blocking and ε-amino acid blocking can be distinguished by using differing pH to when carrying out the reaction.

In the first and second aspects of this invention a sequence specific cleavage reagent is required. When the polypeptides in this aspect have been treated with an alkenyl sulphone reagent, which prevents cleavage by Lys-C at these modified residues, alternative cleavage reagents should be used. Trypsin will cleave these modified polypeptides, but only at arginine residues. Similarly one of the widely available Arg-C enzymes will be appropriate. Chemical cleavage may also be applied with this method. A reagent such as cyanogen bromide which cleaves at methionine residues is appropriate. Chemical cleavage may be advantageous because protease inhibitors may be used during the isolation of the sample of polypeptides from its biological source. The use of protease inhibitors will reduce non-specific degradation of the sample by endogenous proteases.

*Fractionating proteins and peptides*

The methods of this invention can be used to profile populations of proteins generated in numerous ways. Various fractionation techniques exist to sub-sort proteins on the basis of certain features. A population of proteins extracted from a mammalian tissue, for example, is going to contain a significant number of distinct protein species. It is thought there are of the order of 10000 genes expressed in the average human cell, and so as many proteins are expected to be present in a particular tissue. It may be desirable to fractionate these proteins prior to treatment according to this invention. It may also be desirable to fractionate the terminal peptides isolated from a population of proteins using the methods of this invention prior to further manipulations or analysis.

Fractionation steps can be used to reduce the complexity of a population of proteins by resolving a protein population into a number of discrete subsets. Preferably subsets of a uniform size are desirable. This is most readily achieved by separation on the basis of global properties of proteins, that vary over a broad and continuous range, such as size and surface charge. These are the properties used most effectively in 2-D gel electrophoresis. Such separations can be achieved more rapidly than gel electrophoresis using liquid chromatographic techniques. By following one liquid chromatography separation by another, a population of proteins can be resolved to an arbitrary degree, although a large number of sequential chromatographic separation steps could result in sample loss or other artefacts due to non-specific adhesion of proteins or peptides to different chromatographic matrices.

*Cell fractionation*

Proteins are compartmentalised within their cells. Various techniques are known in the art to fractionate proteins on the basis of their cellular compartments. Fractionation protocols involve various cell lysis techniques such as sonication, detergents or mechanical cell lysis that can be followed by a variety of fractionation techniques, such as centrifugation. Separation into membrane proteins, cytosolic proteins and the major membrane bound subcellular compartments, such as the nucleus and mitochondria, is standard practice. Thus certain classes of protein may be effectively ignored or can be specifically analysed. This form of fractionation may be extremely informative if a particular protein is found in a number of subcellular locations since its location is likely to reveal information about its function.

*Fractionation of whole proteins after extraction*

Since proteins are highly heterogeneous molecules numerous techniques for separation of proteins are available. It is possible to separate proteins on the basis of size, hydrophobicity, surface charge and or by affinity to particular ligands. Separation is effected by an assortment of solid phase matrices derivatised with various functionalities that adhere to and hence slow down the flow of proteins through the column on the basis of specific properties. Matrices derivitised with hydrophobic moieties can be used to separate proteins based on their hydrophobicity, while charged resins can be used to separate proteins on the basis of their charge. In a typical chromatographic separation, analyte molecules are injected into columns packed with these a derivitised resin in a loading buffer or solvent that favours adhesion to the solid phase matrix. This is followed by washing the column with steadily increasing quantities of a second buffer or solvent favouring elution. In this way the proteins with the weakest interactions with a given matrix elute first.

It is desirable, after isolation of terminal peptides using the methods of this invention, to analyse the resultant peptides. Fractionation of the terminal peptides generated by the methods of this invention is optional but in populations comprising large numbers of peptides, detection and identification of peptides is greatly facilitated by analytical separation steps. Various liquid chromatography techniques have been used for peptide separations. A preferred technique is High Pressure Liquid Chromatography (HPLC) as this technique combines rapid separation of small volumes of analyte solution whilst also achieving very good resolution of peptides. In HPLC the matrix is designed to be highly incompressible allowing chromatographic separation to be performed at extremely high pressures, which favours rapid and discrete separation. These features make HPLC very attractive for use with mass spectrometry, which is a preferred detection technology for use with peptides. Liquid chromatography mass spectrometry (LCMS) is a well developed field. HPLC systems in-line with electrospray mass spectrometers are in widespread use. HPLC is a fast and effective way of resolving peptide samples generated by the methods of this invention.

Other fractionation procedure may be used as part of the analysis of a population of terminal peptides prior to mass spectrometry depending on the configuration of the mass spectrometer used. Sorting peptides by ion exchange chromatography, for example, may be advantageous, in that short peptides could be separated in an almost sequence dependent manner: the amino acids that are ionisable have known pKa values and hence elution of peptides from such a column at a specific pH, would be indicative of the presence of particular amino acids in that sequence. For example, aspartate residues have a pKa of 3.9 and glutamate residues 4.3. Elution of a peptide at pH 4.3 would be indicative of the presence of glutamate in the peptide. These effects are sometimes masked in large proteins but should

be more distinct in short peptides. Fractions could be analysed by spotting onto a target for subsequent analysis by laser desorption analysis (discussed later in the text). Alternatively an 'autosampler' can be used to inject fractions from chromatographic separations into an electrospray ionisation mass spectrometer system.

*Fractionation by affinity*

A population of proteins can be fractionated by affinity methods. This sort of fractionation method relies on specific interactions between proteins, or classes of proteins, with specific ligands.

Many proteins, for example, exist as complexes with other proteins and analysis of such complexes is often difficult. A cloned protein that is a putative member of a complex can be used to generate an affinity column with the cloned protein acting as an affinity ligand to capture other proteins that normally bind to it. This invention is eminently suited to the analysis of such captured protein complexes.

*Isolation of post-translationally modified proteins*

A large number of affinity ligands are available commercially for specific applications such as the isolation of proteins with post-translational modifications. A number of tagging procedures are also known by which affinity tags such as biotin can be introduced into proteins that have specific post-translational modifications allowing such proteins to be captured using biotin-avidin affinity chromatography.

*Isolation of carbohydrate-modified proteins*

Carbohydrates are often present as a post-translational modification of proteins. Various affinity chromatography techniques for the isolation of these sorts of proteins are known (For a review see Gerard C., Methods Enzymol. 182, 529-539, 'Purification of glycoproteins.' 1990). A variety of natural protein receptors for carbohydrates are known. The members of this class of receptors, known as lectins, are highly selective for particular carbohydrate functionalities. Affinity columns derivitised with specific lectins can be used to isolate proteins with particular carbohydrate modifications, whilst affinity columns comprising a variety of different lectins could be used to isolate populations of proteins with a variety of different carbohydrate modifications. Many carbohydrates have cis-diol groups present. Cis-diols will react with boronic acid derivative to form cyclic esters. This reaction is favoured at basic pH but is easily reversed at acid pH. Resin immobilised derivatives of phenyl boronic acid have been used as ligands for affinity capture of proteins with cis-diol containing carbohydrates. Cis-diols can also be converted into carbonyl groups by oxidation with periodate. These carbonyl groups can be tagged allowing proteins bearing such modifications to be detected or isolated. Biocytin hydrazide (Pierce & Warriner Ltd., Chester, UK) will react with carbonyl groups in periodate-treated carbohydrate species (E.A. Bayer et al. , Anal. Biochem. 170, 271-281, "Biocytin hydrazide - a selective label for sialic acids, galactose, and other sugars in glycoconjugates using avidin biotin technology", 1988). Proteins bearing cis-diol containing carbohydrate modifications in a complex mixture can thus be biotinylated. Biotinylated, hence carbohydrate modified, proteins may then be isolated using an avidinated solid support.

*Isolation of phosphorylated proteins*

A number of research groups have reported on the production of antibodies, which bind to phosphotyrosine residues in a wide variety of proteins (see for example A.R. Frackelton *et al.*, Methods Enzymol 201, 79-92, 'Generation of monoclonal antibodies against phosphotyrosine and their use for affinity purification of phosphotyrosine-containing proteins.', 1991 and other articles in this issue of Methods Enzymol.). This means that a significant proportion of proteins that have been post-translationally modified by tyrosine phosphorylation may be isolated by affinity chromatography using these antibodies as the affinity column ligand.

These phosphotyrosine binding antibodies can be used in the context of this invention to isolate terminal peptides from proteins containing phosphotyrosine residues. The tyrosine-phosphorylated proteins in a complex mixture may be isolated using anti-phosphotyrosine antibody affinity columns. The N-terminal peptides from the fractionated mixture of phosphoproteins may then be isolated according to the methods of this invention.

Techniques for the analysis of phosphoserine and phosphothreonine containing peptides are also known. One class of such methods is based a well known reaction for beta-elimination of phosphates. This reaction results in phosphoserine and phosphothreonine forming dehydroalanine and methyldehydroalanine, both of which are Michael acceptors and will react with thiols. This has been used to introduce hydrophobic groups for affinity chromatography (See for example Holmes C.F., FEBS Lett. 215(1), 21-24, 'A new method for the selective isolation of phosphoserine-containing peptides.' 1987). Dithiol linkers have also been used to introduce fluorescein and biotin into phosphoserine and phosphothreonine containing peptides (Fadden P, Haystead TA, Anal Biochem ***225***(1), 81-8, 'Quantitative and

selective fluorophore labelling of phosphoserine on peptides and proteins: characterization at the attomole level by capillary electrophoresis and laser-induced fluorescence.' 1995; Yoshida O. et al., Nature Biotech 19, 379-382, 'Enrichment analysis of phosphorylated proteins as a tool for probing the phosphoproteome', 2001). The use of biotin for affinity enrichment of proteins phosphorylated at serine and threonine could be used with the methods of this invention so that only the terminal peptides need to be analysed. Similarly anti-fluorescein antibodies are known which would allow fluorescein tagged peptides to be selectively isolated with affinity chromatography. This could be followed by terminal peptide isolation according to the methods of this invention.

A chemical procedure for the isolation of phosphoproteins has also been published (Zhou H. *et al.*, Nature Biotech. 19, 375-378, 'A systematic approach to the analysis of protein phosphorylation', 2001). This procedure relies on the fact that phosphoramidates hydrolyse easily under acid conditions. The procedure involves capping all free amines in a mixture of proteins, followed by blocking all free phosphates and carboxyl groups by coupling the phosphates and carboxyls with a capping group containing an amine functionality to form the corresponding phosphoramidates and amides. The blocked proteins are then treated with acid to unblock the phosphates. The peptides are then reacted with a second amine reagent carrying a protected thiol. This step blocks the phosphates again. The protected thiol was deprotected and used to capture the phosphopeptides selectively onto a thiol reactive resin. These peptides could then be released by acid hydrolysis, after thorough washing of the resin. This procedure is claimed to be applicable to all phosphate groups but phosphotyrosine is acid labile and so the method is unlikely to applicable to phosphotyrosine.

*Other post-translational modifications of proteins*

Proteins that have been modified by ubiquitination, lipoylation and other post-translational modifications may also be isolated or enriched by chromatographic techniques (Gibson J.C., Rubinstein A., Ginsberg H.N. & Brown W. V., Methods Enzymol 129, 186-198, 'Isolation of apolipoprotein E-containing lipoproteins by immunoaffinity chromatography.' 1986; Tadey T. & Purdy W.C. J. Chromatogr. B. Biomed. Appl. 671(1-2), 237-253, 'Chromatographic techniques for the isolation and purification of lipoproteins.' 1995) or affinity ligand based techniques such as immunoprecipitation (Hershko A., Eytan E., Ciechanover A. & Haas A.L., J. Biol. Chem. 257(23), 13964-13970, 'Immunochemical analysis of the turnover of ubiquitin-protein conjugates in intact cells. Relationship to the breakdown of abnormal proteins.' 1982). Populations of proteins with these modifications can all be analysed by the methods of this invention.

The analysis of peptides using mass spectrometry will now be discussed in more detail.

The essential features of a mass spectrometer are as follows:

Inlet System → Ion Source → Mass Analyser → Ion Detector → Data Capture System

There are certain preferred inlet systems, ion sources and mass analysers for the purposes of analysing peptides.

*Inlet systems*

In all of the aspects of this invention a chromatographic or electrophoretic separation may be used to reduce the complexity of the sample prior to analysis by mass spectrometry. A variety of mass spectrometry techniques are compatible with separation technologies particularly capillary zone electrophoresis and High Performance Liquid Chromatography (HPLC). The choice of ionisation source may be limited to some extent if a separation is required as ionisation techniques such as MALDI and FAB (discussed below), which ablate material from a solid surface are less suited to chromatographic separations. It is difficult to link a chromatographic separation in-line with mass spectrometric analysis by one of these techniques. Dynamic FAB and ionisation techniques based on spraying such as electrospray, thermospray and APCI are all compatible with in-line chromatographic separations.

*Ionisation techniques*

For many biological mass spectrometry applications so called 'soft' ionisation techniques are used. These allow large molecules such as proteins and nucleic acids to be ionised essentially intact. The liquid phase techniques allow large biomolecules to enter the mass spectrometer in solutions with mild pH and at low concentrations. A number of techniques are appropriate for use with this invention including but not limited to Electrospray Ionisation Mass Spectrometry (ESI-MS), Fast Atom Bombardment (FAB), Matrix Assisted Laser Desorption Ionisation Mass Spectrometry (MALDI MS) and Atmospheric Pressure Chemical Ionisation Mass Spectrometry (APCI-MS).

*Electrospray Ionisation*

Electrospray ionisation requires that the dilute solution of the analyte molecule is 'atomised' into the spectrometer, i.e. injected as a fine spray. The solution is, for example, sprayed from the tip of a charged needle in a stream of dry nitrogen and an electrostatic field. The mechanism of ionisation is not fully understood but is thought to work broadly as follows. In a stream of nitrogen the solvent is evaporated. With a small droplet, this results in concentration of the analyte molecule. Given that most biomolecules have a net charge this increases the electrostatic repulsion of the dissolved molecule. As evaporation continues this repulsion ultimately becomes greater than the surface tension of the droplet and the droplet disintegrates into smaller droplets. This process is sometimes referred to as a 'Coulombic explosion'. The electrostatic field helps to further overcome the surface tension of the droplets and assists in the spraying process. The evaporation continues from the smaller droplets which, in turn, explode iteratively until essentially the biomolecules are in the vapour phase, as is all the solvent. This technique is of particular importance in the use of mass labels in that the technique imparts a relatively small amount of energy to ions in the ionisation process and the energy distribution within a population tends to fall in a narrower range when compared with other techniques. The ions are accelerated out of the ionisation chamber by the use of electric fields that are set up by appropriately positioned electrodes. The polarity of the fields may be altered to extract either negative or positive ions. The potential difference between these electrodes determines whether positive or negative ions pass into the mass analyser and also the kinetic energy with which these ions enter the mass spectrometer. This is of significance when considering fragmentation of ions in the mass spectrometer. The more energy imparted to a population of ions the more likely it is that fragmentation will occur through collision of analyte molecules with the bath gas present in the source. By adjusting the electric field used to accelerate ions from the ionisation chamber it is possible to control the fragmentation of ions. This is advantageous when fragmentation of ions is to be used as a means of removing tags from a labelled biomolecule.

*Matrix Assisted Laser Desorption Ionisation (MALDI)*

MALDI requires that the biomolecule solution be embedded in a large molar excess of a photo-excitable 'matrix'. The application of laser light of the appropriate frequency results in the excitation of the matrix which in turn leads to rapid evaporation of the matrix along with its entrapped biomolecule. Proton transfer from the acidic matrix to the biomolecule gives rise to protonated forms of the biomolecule which can be detected by positive ion mass spectrometry. This technique imparts a significant quantity of translational energy to ions, but tends not to induce excessive fragmentation despite this. Accelerating voltages can again be used to control fragmentation with this technique though.

*Fast Atom Bombardment*

Fast Atom Bombardment has come to describe a number of techniques for vaporising and ionising relatively involatile molecules. The essential principal of these techniques is that samples are desorbed from surfaces by collision of the sample with accelerated atoms or ions, usually xenon atoms or caesium ions. The samples may be coated onto a solid surface as for MALDI but without the requirement of complex matrices. These techniques are also compatible with liquid phase inlet systems - the liquid eluting from a capillary electrophoresis inlet or a high pressure liquid chromatography system pass through a frit, essentially coating the surface of the frit with analyte solution which can be ionised from the frit surface by atom bombardment.

*Mass Analysers*

In most cases mass determination of each peptide will be sufficient to identify the protein from which the peptide was derived. Mass determination can be performed quite economically by using one of a number of simple mass analyser geometries such as Time Of Flight, Quadrupole and Ion Trap instruments. Fragmentation of peptides by collision induced dissociation can be used to identify proteins whose identity is not determined by the mass of its terminal peptides alone. More complex mass analyser geometries may be necessary if more information about a peptide is required, although ion traps may be sufficient for this purpose as well.

*MS/MS and $MS^n$ analysis of peptides*

Tandem mass spectrometers allow ions with a pre-determined mass-to-charge ratio to be selected and fragmented by collision induced dissociation (CID). The fragments can then be detected providing structural information about the selected ion. When peptides are analysed by CID in a tandem mass spectrometer, characteristic cleavage patterns are observed, which allow the sequence of the peptide to be determined. Natural peptides typically fragment randomly at the amide bonds of the peptide backbone to give series of ions that are characteristic of the peptide. CID

fragment series are denoted $a_n$, $b_n$, $c_n$, etc. for cleavage at the $n^{th}$ peptide bond where the charge of the ion is retained on the N-terminal fragment of the ion. Similarly, fragment series are denoted $x_n$, $y_n$, $z_n$, etc. where the charge is retained on the C-terminal fragment of the ion.

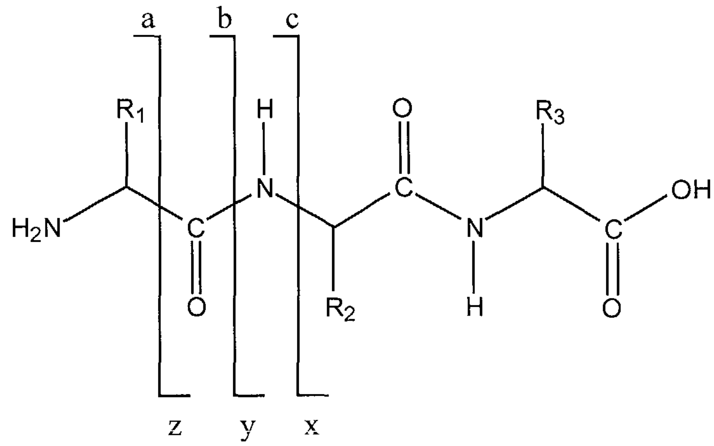

Trypsin and thrombin are favoured cleavage agents for tandem mass spectrometry as they produce peptides with basic groups at both ends of the molecule, i.e. the alpha-amino group at the N-terminus and lysine or arginine side-chains at the C-terminus. This favours the formation of doubly charged ions, in which the charged centres are at opposite termini of the molecule. These doubly charged ions produce both C-terminal and N-terminal ion series after CID. This assists in determining the sequence of the peptide. Generally speaking only one or two of the possible ion series are observed in the CID spectra of a given peptide. In low-energy collisions typical of quadrupole based instruments the b-series of N-terminal fragments or the y-series of C-terminal fragments predominate. If doubly charged ions are analysed then both series are often detected. In general, the y-series ions predominate over the b-series.

A typical tandem mass spectrometer geometry is a triple quadrupole which comprises two quadrupole mass analysers separated by a collision chamber, also a quadrupole. This collision quadrupole acts as an ion guide between the two mass analyser quadrupoles into which a gas can be introduced to allow collision with the ion stream from the first mass analyser. The first mass analyser selects ions on the basis of their mass/charge ration which pass through the collision cell where they fragment. The degree of fragmentation may be controlled by varying either the electric fields used to accelerate the ions or by varying the gas in the collision cell, e.g. helium can be replaced by neon. The fragment ions are separated and detected in the third quadrupole. Induced cleavage can be performed in geometries other than tandem analysers. Ion traps mass spectrometers can promote fragmentation through introduction of a gas into the trap itself with which trapped ions can collide after acceleration. Ion traps generally contain a bath gas, such as helium but addition of neon for example, promotes fragmentation. Similarly photon induced fragmentation could be applied to trapped ions. Another favourable geometry is a Quadrupole/Orthogonal Time of Flight tandem instrument where the high scanning rate of a quadrupole is coupled to the greater sensitivity of a reflectron TOF mass analyser to identify the products of fragmentation.

Conventional 'sector' instruments are another common geometry used in tandem mass spectrometry. A sector mass analyser comprises two separate 'sectors', an electric sector which focuses an ion beam leaving a source into a stream of ions with the same kinetic energy using electric fields. The magnetic sector separates the ions on the basis of their mass to generate a spectrum at a detector. For tandem mass spectrometry a two sector mass analyser of this kind can be used where the electric sector provide the first mass analyser stage, the magnetic sector provides the second mass analyser, with a collision cell placed between the two sectors. This geometry might be quite effective for cleaving labels from a mass labelled nucleic acid. Two complete sector mass analysers separated by a collision cell can also be used for analysis of mass labelled nucleic acids.

*Ion Traps*

Ion Trap mass spectrometers are a relative of the quadrupole spectrometer. The ion trap generally has a 3 electrode construction - a cylindrical electrode with 'cap' electrodes at each end forming a cavity. A sinusoidal radio frequency potential is applied to the cylindrical electrode while the cap electrodes are biased with DC or AC potentials. Ions injected into the cavity are constrained to a stable trajectory within the trap by the oscillating electric field of the cylindrical electrode. However, for a given amplitude of the oscillating potential, certain ions will have an unstable

trajectory and will be ejected from the trap. A sample of ions injected into the trap can be sequentially ejected from the trap according to their mass/charge ratio by altering the oscillating radio frequency potential. The ejected ions can then be detected allowing a mass spectrum to be produced.

Ion traps are generally operated with a small quantity of a 'bath gas', such as helium, present in the ion trap cavity. This increases both the resolution and the sensitivity of the device as the ions entering the trap are essentially cooled to the ambient temperature of the bath gas through collision with the bath gas. Collisions both increase ionisation when a sample is introduced into the trap and dampen the amplitude and velocity of ion trajectories keeping them nearer the centre of the trap. This means that when the oscillating potential is changed, ions whose trajectories become unstable gain energy more rapidly, relative to the damped circulating ions and exit the trap in a tighter bunch giving a narrower larger peaks.

Ion traps can mimic tandem mass spectrometer geometries, in fact they can mimic multiple mass spectrometer geometries allowing complex analyses of trapped ions. A single species of selected mass-to-charge ratio from a sample can be retained in a trap, i.e. all other species can be ejected. The retained species can be excited by super-imposing a second oscillating frequency on the first. The excited ions will then collide with the bath gas and will fragment if sufficiently excited. The resultant fragments can then be analysed further. It is possible to retain a fragment ion for further analysis by ejecting unwanted ions from the trap. The retained fragment may be excited again to induce further fragmentation. This process can be repeated for as long as sufficient sample exists to permit further analysis. It should be noted that these instruments generally retain a high proportion of fragment ions after induced fragmentation. These instruments and FTICR mass spectrometers (discussed below) represent a form of temporally resolved tandem mass spectrometry rather than spatially resolved tandem mass spectrometry which is found in linear mass spectrometers.

*Fourier Transform Ion Cyclotron Resonance Mass Spectrometry (FTICR MS)*

FTICR mass spectrometers have similar features to ion traps in that a sample of ions is retained within a cavity but in FTICR MS the ions are trapped in a high vacuum chamber by crossed electric and magnetic fields. The electric field is generated by a pair of plate electrodes that form two sides of a box. The box is contained in the field of a magnet, which in conjunction with the two electric field-generating plates, referred to as the trapping plates, constrain injected ions to a stable cycloidal trajectory between the trapping plates, perpendicular to the applied magnetic field. The ions are excited into wider orbits by applying a radio-frequency pulse to two 'transmitter plates' which form two further opposing sides of the box. The cycloidal motions of the ions generate corresponding electric fields in the remaining two opposing sides of the box which comprise the 'receiver plates'. The excitation pulses excite ions to larger orbits which decay as the coherent motions of the ions is lost through collisions. The corresponding signals detected by the receiver plates are converted to a mass spectrum by Fourier transform analysis.

For induced fragmentation experiments these instruments can perform in a similar manner to an ion trap - all ions except a single species of interest can be ejected from the trap. A collision gas can be introduced into the trap and fragmentation can be induced. The fragment ions can be subsequently analysed. Generally fragmentation products and bath gas combine to give poor resolution if analysed by FT of signals detected by the 'receiver plates', however the fragment ions can be ejected from the cavity and analysed in a tandem configuration with a quadrupole, for example.

## Examples

<u>Example 1 - Synthesis of propenyl and isobutenyl sulphones</u>

*Synthesis of phenyl-1-propenylsulphone*

Synthesis of 1-chloro-2-propanol: it was found that 1-chloro-2-propanol from commercial sources is only available as a mixture of two isomers: 1-chloro-2-propanol and 2-chloro-1-propanol. Pure 1-Chloro-2-propanol was synthesised by the method disclosed by Stewart C.A. and Calvin Van der Werf A., J. Am. Chem. Soc. 76, 1259-64 (1954). Lithium aluminium hydride (10g, 0.256 mol) is added portion-wise with stirring to a round-bottomed two-necked flask containing 200 ml of ice cooled absolute ether. The flask is connected to a condenser and a separation funnel. 66g (0.256 mol) of chloroacetone was added over a period of 90 minutes. The reaction mixture was stirred for one hour after complete addition of the chloroacetone. This was followed by the decomposition of the hydride with water. 100 ml of 4N sulphuric acid were then added to give a separable mixture. The ether layer was separated and the aqueous layer was extracted three times with ether. The combined ether extracts were washed with water, and dried over magnesium sulphate. The ether was removed at room temperature by rotary evaporation. The residual oil was fractionally distilled using a water pump. A first fraction collected at 31°C (9mm Hg) was mainly ether. The second fraction, collected at 38°C (5mm Hg), was 1-chloro-2-propanol, which was obtained as a colourless oil (yield 32.4g; 49%). Analysis by $^1H$ NMR confirmed that the correct and pure isomer was obtained.

Synthesis of phenyl-2-(hydroxypropyl)sulphide: 11 g (0.1 mol) of benzenethiol and 9.45g (0.1 mol) of 1-chloro-2-propanol in 50 ml of 2-propanol were added to 75 ml of a 1N solution of potassium hydroxide in 2-propanol. To this solution 7.2g (2 x 0.1 mol) of $NaBH_4$ was added with stirring. The reaction mixture was stirred for 24 hours at room temperature, however TLC showed that there was product formation after only a short time. The heterogeneous reaction mixture was extracted with ether, washed with water and dried (sodium sulphate). After evaporation of the solvent, the residue was eluted from a silica column with ethyl acetate/n-hexane (50/50 v/v). This afforded 2-hydroxypropylphenylsulphide as a colourless oil, (yield 15.45g, 92 %).

Conversion of phenyl-2-(hydroxypropyl)sulphide to phenyl-2-(chloropropyl)sulphide: 9.63g (0.081 mol) of thionyl chloride was added drop-wise to 9.1g (0.054 mol) of phenyl-2-(hydroxypropyl)sulphide in 70 ml of absolute chloroform. The reaction was stirred at room temperature for 24 hours. After completion of the reaction, the solvent was evaporated and the residue was eluted from a silica column with an ethyl acetate/n-hexane solvent system (50:50 v/v) to yield the corresponding phenyl-2-(chloropropyl)sulphide: as a colourless oil (Yield 9.51g, 95 %).

Conversion of phenyl-2-(chloropropyl)sulphide to phenyl-2-(chloropropyl)sulphone: 7.5g (0.04 mol.) of phenyl-2-(chloropropyl)sulphide was heated in 400 ml glacial acetic acid with 50 ml of a 30% solution of $H_2O_2$ for 2h, under reflux. The reaction mixture then poured into cold water (200ml), but the product was not crystallised after cooling. The product then extracted by ether, washed with water and dried on sodium sulphate. The residue after evaporation was eluted from a silica gel column with ethyl acetate/n-hexane (50/50 v/v) to yield phenyl-2-(chloropropyl)sulphone as a colourless oil (yield 7.9g, 91 %).

Conversion of phenyl-2-(chloropropyl) sulphone to phenyl-1-propenyl sulphone: 5.6g (0.025 mol) of phenyl-2-chloropropyl sulphone was dissolved in 50 ml of THF and then treated with 4.06g (0.04mol) of triethylamine. The reaction was stirred overnight at room temperature. The residue after evaporation of the solvent was eluted from a silica gel column with ethyl acetate/n-hexane (50/50 v/v) to yield a residual oil corresponding to the cis-trans forms of phenyl-2propenyl sulphone (yield 4.18g, 92%). The oily product was then crystallised from ether/petroleum ether to yield 2g of the trans-form of phenyl-1-propenyl sulphone as colourless crystals.

*Synthesis of phenyl-1-isobutenyl sulphone*

Pure isomer of 1-chloro-2-methyl-2-propanol is available from Sigma-Aldrich, Dorset, UK. Purity of the isomer was confirmed by 'H NMR.

Synthesis of phenyl-2-methyl-2-(hydroxypropyl) sulphide: 22g (0.2 mol) of benzenethiol and 18.9g (0.2 mol) of 1-chloro-2-methyl-2-propanol in 100 ml of 2-propanol was added to 75ml, 1N solution of potassium hydroxide in 2-propanol. 14.2g (2 x 0.2 mol) of $NaBH_4$ was added to this solution with stirring. The reaction mixture was stirred for 24 hours at room temperature, however TLC showed, product formation began after only a short time. The heterogeneous reaction mixture was extracted with ether, washed with water and dried (sodium sulphate). The residue, after evaporation of the solvent was eluted from a silica gel column with ethyl acetate/n-hexane (50/50 v/v), which afforded phenyl-2-methyl-2-hydroxypropylsulphide as colourless oil ($C_{10}H_{14}OS$, Mr = 182, yield 32.75g, 90 %).

Conversion of phenyl-2-hydroxy-2-methylpropyl-sulphide to phenyl-2-chloro-2-methylpropylsulphide: 117.8g (0.15 mol) of thionyl chloride was added drop-wise to 13.5g (0.075mol) of phenyl-2-hydroxy-2-methylpropylsulphide in 75 ml of absolute chloroform. The reaction was stirred at room temperature for 24 hours. After completion of the reaction, the solvent was evaporated and the residue was eluted from a silica gel column with an ethyl acetate/n-hexane solvent system (50:50 v/v) to yield the corresponding phenyl-2-chloro-2-methylpropyl-sulphide as a colourless oil ($C_{10}H_{13}ClS$, Mr = 200.5, yield 26.76g, 89 %).

Conversion of phenyl-2-Chloro-2-methylpropyl-sulphide to phenyl-2-chloro-2-methylpropylsulphone: 15.5g (0.077 mol) of phenyl-2-chloro-2-methylpropyl-sulphone was heated in 40 ml glacial acetic acid with 70 ml of a 30 % solution of $H_2O_2$ for 2 hours, under reflux. The reaction mixture was then poured into cold water (200ml). The product did not crystallise after cooling overnight. The product was then extracted in ether, washed with water and finally dried over sodium sulphate to yield crude phenyl-2-chloro-2-methylpropylsulphone as colourless oil 15.33g ($C_{10}H_{13}ClO_2S$, Molecular Mass = 232.5). 0.7g of this crude product was kept for further chromatographic purification, while the rest of the product was converted to phenyl-1-isobutenyl sulphone as described in the next section.

Conversion of phenyl-2-chloro-2-methylpropylsulphone to phenyl-1-isobutenyl sulphone: 14.6g (0.062 mol) of phenyl-2-chloro-2-methylpropyl sulphone was dissolved in 60 ml of tetrahydrofuran (THF) and then treated with 12.52g (0.124 mol) of triethylamine. The reaction was stirred overnight at room temperature to give phenyl-1-isobutenyl sulphone.

*Synthesis of pyridyl-1-propenylsulphone*

Preparation of pyridine-3-sulphonylchloride: 3.18g (0.02 mol) of pyridine-3-sulphonic acid ($C_5H_5NSO_3$) was mixed with 8.34g (0.04 mol) of $PCl_5$ in a dry flask. The flask was protected from moisture and heated at 130-140°C

under reflux with stirring for 2 hours. The reaction mixture was then cooled. The cold solidified reaction mixture was then triturated with $CHCl_3$ to remove $PCl_5$ and $POCl_3$. The supernatant liquid was discarded. The triturating process was repeated using fresh $CHCl_3$ and the product was finally triturated with $CHCl_3$ saturated with hydrogen chloride. The hydrogen chloride was prepared by the slow addition of concentrated sulphuric acid ($H_2SO_4$) from a dropping funnel to sodium chloride in a round bottom flask. The round bottom flask was connected to the trituration reaction vessel by rubber tubing. A white powder formed, which was filtered, washed with $CHCl_3$ and finally dried in a vacuum. This process gave 3-pyridinesulphonylchloride-HCl (yield 3.05g, 85 %) $C_5H_4NSO_2Cl$, (Melting point: 141-143°C). This procedure is described by Reinhart F. E., J. Franklin. Ind. 236, 316-320 (1943).

Preparation of pyridine-3-(2-hydroxypropyl)sulphone: into a boiling solution of 3.52g (0.028 mol) $Na_2SO_3$ and 4.36g (0.052 mol) $NaHCO_3$ in 50 ml water, the 3-pyridinesulphonyl chloride hydrochloride 2.828 g (0.014 mol) was added portion wise. After completion of addition, it was heated for a further 5 minutes, filtered and the filtrate evaporated to dryness. The fully pulverised residue was suspended in 100 ml of absolute dimethylformamide and heated with 1 g (3 mmol.) of tetrabutylammonium bromide (serves as a transfer catalyst) and 2.22 g (0.028 mol) of 1-chloro-2-propanol, prepared as described above. The reaction mixture was refluxed for 24 hours. After filtration of the solid, the filtrate was evaporated to dryness, and the residue oil was eluted from a silica gel column with ethyl acetate and methanol (80/20 v/v).

Mesylation of pyridine-3-(2-hydroxypropyl)sulphone and elimination of mesylated hydroxyl to give pyridine-1-propenylsulphone: a mixture of 2.0 g (0.00995 mol) of pyridine-3-(2-hydroxypropyl) sulphone in 25 ml tetrahydrofuran (THF) and triethylamine 2.0 g (0.0199 mol) was cooled to 0°C. To this was added 2.23 g (0.0149 mol) of methane sulphonyl chloride. The reaction mixture was stirred for 6 hours at 0°C followed by stirring for 6 hours at room temperature. The precipitate of triethylammonium chloride was filtered off and the solvent was evaporated. The residual oil was then treated with 1.5 g (0.0149 mol) of triethylamine and left stirring for 48 hours at room temperature. 25 ml of THF was then added, and the precipitate was filtered off. After evaporation of the solvent, the residue was eluted from a silica gel column with a solvent comprising 75 % ethyl acetate and 25 % n-hexane to afford a colourless oil, which solidified on cooling to give 1.5 g of pyridine-1-propenylsulphone (83 % yield).

*Synthesis of pyridyl-1-isobutenylsulphone*

Preparation pyridine-3-(2-hydroxyisobutyl)sulphone: pyridine-3-sulphonylchloride was prepared as described above. 23 g (0.108 mol) of pyridine-3-sulphonyl chloride hydrochloride was added portion-wise to a boiling solution of 21.33g (0.169 mol) $Na_2SO_3$ and 25.34 g (0.3 mol) $NaHCO_3$ in 150 ml water. After completion of the addition, the reaction was heated for a further 1 hour, filtered and the filtrate evaporated to dryness. The fully pulverised residue that was obtained was suspended in 100 ml of absolute dry DMF. To this was added 10.9 g (0.11 mol) triethylamine (TEA) and 11.7 g (0.108 mol) of 1-chloro-2-isobutanol (the addition of TEA was used to facilitate removal of the chloride from the 1-chloro-2-isobutanol). The reaction mixture was heated in an oil bath at 100-110°C for about 30 hours. After filtration of the reaction mixture, the filtrate was evaporated to the dryness and the residue was eluted from a silica gel column with a solvent mixture of ethyl acetate 90 % and methanol 10 %. After evaporation of the solvent, the residue was recrystallised from ether as white crystals (yield 12.07 g, 51 %, melting point: 122-123°C).

Mesylation of the pyridine-3-(2-hydroxyisobutyl)sulphone, followed by the elimination of the mesylated hydroxyl to form pyridine-1-isobutenylsulphone: a mixture of 1.4 g (0.0065 mol) of pyridine-3-(2-hydroxyisobutyl)sulphone in 25 ml of THF and 1.81 g of triethylamine (0.018 mol) was cooled to 0°C. To this was added 1.52 g (0.013 mol.) of methane sulphonyl chloride. The reaction mixture was stirred for 24 hours at 0°C. The precipitate of triethylammonium chloride was filtered off and the solvent was evaporated. The residual oil was then treated with 1.81 g triethylamine (0.018 mol), i.e. two molar equivalents, and left stirring for 48 hours at 0°C. After evaporation of the triethylamine, the residue was eluted from a silica gel column with a solvent comprising 75 % ethyl acetate and 25 % n-hexane. Two spots that were close together were isolated and identified. The upper spot corresponding to the unwanted isomer pyridine-3-(2-isobutenyl)sulphone gave 90 mg (7 % yield) as fine white crystals from ether/petroleum ether (melting point: 82-83°C). The second spot corresponding to the required isomer pyridine-3-(1-isobutenyl)sulphone gave 1.013 g (79 % yield) from ether/petroleum ether, (melting point: 50-51°C). (Note: It was found that the desired isomer was obtainable in good yield when the temperature during mesylation with triethylamine is kept around 0°C. As the temperature increases above 0°C during this procedure, the proportion of the unwanted isomer increases.)

Example 2 - Reactions of dipeptides and peptides with lysine-selective reagents

Reactions of a number of dipeptides and peptides with various tags were carried out in order to determine the relative reactivities and selectivities of the tags. In particular, it was the aim of this series of experiments to determine which tags show the greatest discrimination against alpha amino-groups whilst still reacting selectively with epsilon amino groups.

Reaction Conditions

1. In all cases the labelling was carried out in 0.1 M borate buffer, at pH ~ 9.5
2. Various labelling times were used, and conducted at either room temperature (RT) or on ice (0°C).
3. For most of the labels, 250 nmol of substrate were reacted with 2 μmol of label in 10 μl of 50:50 acetonitrile: borate buffer. This gives an 8-fold excess of label to substrate although some substrates had 2 reaction sites and so there would have been only a 4-fold excess of label in these reactions.
4. Phenyl isobutenyl sulphone and phenyl styryl sulphone were not soluble in the above solvent mixture. An additional 10 μl of acetonitrile was added to dissolve these tags so the final volume for these two experiments was doubled.

Table 2 -

| Dipeptides | | | |
|---|---|---|---|
| **Dipeptides** | **Full Name** | **Mass** | **$(M+H)^+$** |
| **AL** | Alanine-leucine | 202.2 | 203.2 |
| **VA** | Valine-alanine | 188.2 | 189.2 |
| **GL** | Glycine-leucine | 188.2 | 189.2 |
| **GK** | Glycine-lysine | 203.7 | 204.7 |
| **VK** | Valine-lysine | 245.8 | 246.8 |
| **PG** | Proline-glycine | 172.2 | 173.2 |
| **GH** | Glycine-histidine | 212.2 | 213.2 |

Table 3 -

| Peptides | | | |
|---|---|---|---|
| **Peptides** | **Full Name** | **Mass** | **$(M+H)^+$** |
| **N-Formyl Peptide** | N-Formyl-norleucine-leucine-phenylalanine-norleucine-tyrosine-lysine | 824 | 825 |
| **PFGK** | Proline-phenylalanine-glycine-lysine | 447.5 | 448.5 |
| **VGSE** | Valine-glycine-serine-glutamic acid | 390.4 | 391.4 |

Table 4 -

| Tags | |
|---|---|
| **Tag** | **Name** |
| **NEM** | N-ethyl maleimide |
| **PTS** | Phenyl trifluoropropenyl sulphone |
| **PPS** | Phenyl-1-propenyl sulphone |
| **PyPS** | Pyridyl-1-propenyl sulphone |
| **PIBS** | Phenyl isobutenyl sulphone |
| **PyIBS** | Pyridyl-isobutenyl-sulphone |
| **PSS** | Phenyl trans styryl sulphone |

*Analysis*

In each case, products were separated from unincorporated label by Thin Layer Chromatography (TLC) developed in diethyl ether. Products were extracted from the silica with water and 50 % acetonitrile and evaporated to dryness. After re-suspension in 50 % ACN:$H_2O$ products were analysed by ES-MS.

Rather than include all of the spectra for these experiments, the peak heights of the key peaks in the spectra were measured and expressed as a percentage of the total. These results thus give a simplified indication of the peak heights in the original mass spectra representing each significant species, i.e. species that are unlabelled or have 1, 2 or 3 labels. In cases where the $(M+Na)^+$ peak for a given species was substantial, this was included as part of the peak height of the $(M+H)^+$ species. In most cases there were no significant peaks that did not correspond to peptides or labelled species. The results are shown in the tables that follow.

*Results*

Table 5 -

| Labelling with N-Ethyl Maleimide (125.1) 2.5 hours at room temperature (RT) | | | | |
|---|---|---|---|---|
| **Peptide** | **Number of Labels** | | | |
| | **0** | **1** | **2** | **3** |
| **N-formyl peptide** | 0 | 100 | 0 | 0 |
| **PFGK** | 0 | 6 | 94 | 0 |
| **VGSE** | 81 | 19 | 0 | 0 |

Table 6 -

| 40 min at 0°C | | | | |
|---|---|---|---|---|
| **Peptide** | **Number of Labels** | | | |
| | **0** | **1** | **2** | **3** |
| **N-formyl peptide** | 88 | 12 | 0 | 0 |
| **PFGK** | 12 | 75 | 13 | 0 |
| **VGSE** | 97 | 3 | 0 | 0 |

Table 7 -

| 40 min at 0°C | | | | |
|---|---|---|---|---|
| **Peptide** | **Number of Labels** | | | |
| | **0** | **1** | **2** | **3** |
| **VA** | 75 | 25 | 0 | 0 |
| **GL** | 39 | 61 | 0 | 0 |
| **GK** | 27 | 64 | 9 | 0 |
| **PG** | 83 | 17 | 0 | 0 |
| **GH** | 48 | 52 | 0 | 0 |

This experiment confirms the fact that N-ethyl maleimide reacts selectively with epsilon amino groups in preference to alpha amino groups.

The N-formyl peptide is not very soluble and occasionally precipitated with all tags tested so results with this peptide are quite variable.

Table 8 -

| PSS Labelling (244.32) 96 hr at RT | | | | |
|---|---|---|---|---|
| **Peptide** | **Number of Labels** | | | |
| | **0** | **1** | **2** | **3** |
| **VA** | 100 | 0 | 0 | 0 |
| **GL** | 100 | 0 | 0 | 0 |
| **GK** | 62 | 35 | 0 | 0 |
| **VK** | 74 | 26 | 0 | 0 |

Table 9 -

| PPS Labelling (182) 72 hr at RT | | | | |
|---|---|---|---|---|
| **Peptide** | **Number of Labels** | | | |
| | **0** | **1** | **2** | **3** |
| **VA** | 40 | 60 | 0 | 0 |
| **GL** | 3 | 97 | 0 | 0 |
| **GK** | 0 | 8 | 92 | 0 |
| **VK** | 0 | 58 | 42 | 0 |

Table 10 -

| 48 hr at RT | | | | |
|---|---|---|---|---|
| **Peptide** | **Number of Labels** | | | |
| | **0** | **1** | **2** | **3** |
| **N-formyl peptide** | 0 | 100 | 0 | 0 |
| **PFGK** | 0 | 0 | 100 | 0 |
| **VGSE** | 46 | 54 | 0 | 0 |

Table 11 -

| PIBS Labelling (196) - 72 hr at RT | | | | |
|---|---|---|---|---|
| **Peptide** | **Number of Labels** | | | |
| | **0** | **1** | **2** | **3** |
| **VA** | 95 | 5 | 0 | 0 |
| **GL** | 73 | 27 | 0 | 0 |
| **GK** | 41 | 50 | 9 | 0 |
| **VK** | 43 | 57 | 0 | 0 |

Table 12 -

| 48 hr at RT | | | | |
|---|---|---|---|---|
| **Peptide** | **Number of Labels** | | | |
| | **0** | **1** | **2** | **3** |
| **N-formyl peptide** | 72 | 28 | 0 | 0 |
| **PFGK** | 44 | 56 | 0 | 0 |
| **VGSE** | 100 | 0 | 0 | 0 |

Table 13 -

| PyPS Labelling (183) - 2 hr at RT | | | | |
|---|---|---|---|---|
| **Peptide** | **Number of Labels** | | | |
| | **0** | **1** | **2** | **3** |
| **VA** | 75 | 25 | 0 | 0 |
| **GL** | 44 | 56 | 0 | 0 |
| **GK** | 0 | 38 | 62 | 0 |
| **VK** | 2 | 80 | 18 | 0 |

Table 14 -

| PTS Labelling - 2 hr RT | | | | |
|---|---|---|---|---|
| **Peptide** | **Number of Labels** | | | |
| | **0** | **1** | **2** | **3** |
| **VA** | 100 | 0 | 0 | 0 |
| **GL** | 25 | 75 | 0 | 0 |
| **GK** | 0 | 74 | 26 | 0 |
| **VK** | 0 | 100 | 0 | 0 |

It was believed by the inventors that phenyl propenyl sulphone and pyridyl propenyl sulphone would show a similar degree of selectivity for epsilon-amino groups, with the corresponding phenyl isobutenyl sulphone and pyridyl isobutenyl sulphone showing greater selectivity. It was anticipated by the inventors that the phenyl trifluoropropenyl sulphone would show greater selectivity than the corresponding propenyl sulphones as the trifluoromethyl group is slightly bulkier than the methyl group in these respective reagents. Similarly it was expected that the phenyl propenyl sulphone and pyridyl propenyl sulphone would be more reactive than the corresponding isobutenyl sulphones. The pyridyl compounds were expected to be more reactive than the corresponding phenyl compounds. These expectations are borne out by the results above. The reactivity of the trifluoropropenyl compound was expected to be high and was found to be similar if not greater than the pyridyl propenyl sulphone, whilst having a much greater apparent selectivity. Maleimide has a selectivity which is similar, but slightly lower than the pyridyl propenyl sulphone with a reactivity that is slightly greater. Most of these reagents, with the exception of the N-ethyl maleimide and the isobutenyl reagents, reacted with histidine residues, which should be taken into account in the analysis of the labelled peptides. All of these reagents showed significant reaction with the alpha-amino group of glycine. This is to be expected as the glycine alpha-amino group is the least hindered of the alpha-amino groups and is thus least affected by the hindering groups on the Michael reagents. The glycine alpha-amino group is still intrinsically less nucleophilic than the epsilon-amino group of lysine and the discrimination of all of the above reagents can be improved by careful control of the reaction times and by use of higher pH, i.e. >11 (results not shown).

Example 3 - Experiments using direct injection analysis of reactions

In the experiments carried out to give the following results, all samples, except for PyIBS, were analysed directly by injection of the reaction mixture into an electrospray mass spectrometer, i.e. without TLC clean up and therefore in the presence of excess label. For PyIBS incubations products were separated from unincorporated label by TLC developed in diethyl ether. Products were extracted from the silica with water and 50 % acetonitrile and evaporated to dryness. After re-suspension in 50 % ACN:$H_2O$ products were analysed by ES-MS.

Again, rather than include all of the mass spectra, the relative peak heights of each significant species in the mass spectrum, i.e. unlabelled peptide or peptide with 1 or 2 labels, were measured and expressed as a percentage of the total. These figures are shown in the tables that follow. In cases where the $(M+Na)^+$ peak was substantial, this was included in the $(M+H)^+$ peak. Unreacted label peaks were ignored.

*Reaction Conditions*

In all cases the labelling was carried out in 0.1 M borate buffer at pH ~ 9.5.

Various labelling times were used, and conducted at either room temperature (RT) or on ice (0°C).

625 nmol of each dipeptide was reacted with 20 µmol of label in a total volume of 50 µl of 50:50 acetonitrile: borate buffer. This gives a minimum of 32 fold molar excess of label per molecule of dipeptide. In the cases where two sites were available for labelling this represents a minimum of 16 fold excess per site. The N-formyl peptide is more than 4 times the molecular weight, less was used and there is only one site for labelling (C-terminal lysine), the molar excess in this case is 320 fold.

Table 15 -

| Labelling with N-ethyl maleimide (125.1), 32 x molar excess | | | | | | |
|---|---|---|---|---|---|---|
| **Peptide** | **Number of Labels** | | | | | |
| | *60 min on ice* | | | *40 min on ice* | | |
| | **0** | **1** | **2** | **0** | **1** | **2** |
| **AL** | 22 | 78 | 0 | | | |
| **GL** | 15 | 85 | 0 | | | |
| **VA** | 30 | 70 | 0 | 39 | 61 | 0 |
| **PG** | 70 | 30 | 0 | | | |
| **GK** | 0 | 29 | 71 | | | |
| **VK** | 21 | 27 | 52 | | | |
| **N-formyl peptide** | 4 | 96 | 0 | 17 | 83 | 0 |
| **GH** | 0 | 100 | 0 | | | |

Table 16 -

| Labelling with trifluoro-1-phenyl-sulphonyl-1-propene (PTS) (236.21), 32 x molar excess | | | | | | | | | |
|---|---|---|---|---|---|---|---|---|---|
| **Peptide** | **Number of Labels** | | | | | | | | |
| | *90 min on ice* | | | *60 min on ice* | | | *30 min on ice* | | |
| | **0** | **1** | **2** | **0** | **1** | **2** | **0** | **1** | **2** |
| **AL** | 89 | 11 | 0 | | | | | | |
| **GL** | 61 | 39 | 0 | | | | | | |
| **VA** | 100 | 0 | 0 | | | | | | |
| **PG** | 87 | 13 | 0 | | | | | | |
| **GK** | 17 | 83 | 0 | 14 | 86 | 0 | 27 | 73 | 0 |
| **VK** | 14 | 86 | 0 | | | | | | |

Table 16 - (continued)

| Labelling with trifluoro-1-phenyl-sulphonyl-1-propene (PTS) (236.21), 32 x molar excess | | | | | | | | | |
|---|---|---|---|---|---|---|---|---|---|
| **Peptide** | **Number of Labels** | | | | | | | | |
| | *90 min on ice* | | | *60 min on ice* | | | *30 min on ice* | | |
| | **0** | **1** | **2** | **0** | **1** | **2** | **0** | **1** | **2** |
| **N-FP** | 87 | 13 | 0 | | | | | | |
| **GH** | 25 | 64 | 11 | 21 | 67 | 12 | | | |
| NB. VK at 120 min was 6 94 0<br>N-FP may have come out of solution. | | | | | | | | | |

Table 17 -

| Labelling with pyridyl-isobutenyl-sulphone (PyIBS) (197), 32 x molar excess | | | | | | |
|---|---|---|---|---|---|---|
| **Peptide** | **Number of Labels** | | | | | |
| | *56 hours RT* | | | *40 hours RT* | | |
| | **0** | **1** | **2** | **0** | **1** | **2** |
| **AL** | 78 | 22 | 0 | 81 | 19 | 0 |
| **GL** | 30 | 70 | 0 | 41 | 59 | 0 |
| **VA** | 88 | 12 | 0 | 78 | 22 | 0 |
| **PG** | 84 | 16 | 0 | 84 | 16 | 0 |
| **GK** | 0 | 38 | 62 | 6 | 46 | 48 |
| **VK** | 7 | 87 | 6 | 11 | 85 | 4 |
| **N-formyl peptide** | 15 | 85 | 0 | 24 | 76 | 0 |
| **GH** | 20 | 49 | 29 | 27 | 54 | 19 |

Table 18 -

| PIBS labelling (196), 32 x molar excess - 48 hr at 37°C | | | |
|---|---|---|---|
| **Peptide** | **Number of Labels** | | |
| | **0** | **1** | **2** |
| **AL** | 81 | 19 | 0 |
| **GL** | 61 | 39 | 0 |
| **VA** | 79 | 21 | 0 |
| **PG** | 88 | 12 | 0 |
| **GK** | 32 | 59 | 9 |
| **VK** | 40 | 47 | 13 |
| **N-formyl peptide** | 15 | 85 | 0 |
| **GH** | 67 | 33 | 0 |

Table 19 -

| PSS labelling (244.32), 32 x molar excess - 48 hr at 37°C | | | |
|---|---|---|---|
| **Peptide** | **Number of Labels** | | |
| | **0** | **1** | **2** |
| **AL** | NA | NA | NA |
| **GL** | NA | NA | NA |
| **VA** | NA | NA | NA |
| **PG** | NA | NA | NA |
| **GK** | 42 | 58 | 0 |
| **VK** | 32 | 40 | 28 |
| **N-formyl peptide** | NA | NA | NA |
| **GH** | NA | NA | NA |
| NA not analysed. In view of the GK and VK results, no further samples were analysed. NB precipitation of a white salt was noted even though the ACN was increased. | | | |

As can be seen, the results of the above experiment are largely in agreement with the results of the first set of experiments in this Example.

Example 4 - Determining whether a single hindered Michael reagent on lysine ε-$NH_2$ prevents the addition of a second label.

The following experiments were carried out to confirm that peptides that had been reacted with one label were resistant to further labelling at the labelled site by a second tag.

*Reaction Conditions*

For the two peptides used in these experiments, VK and GK, 250 nmol of peptide were reacted exhaustively with a large excess of label. The labelled peptide was then separated from unreacted label by TLC and the labelled peptide was then recovered from the TLC plate.

The recovered labelled peptides were then reacted with 2 μmol of label in 10 μl of 50:50 acetonitrile:borate buffer. This gives a 4-fold excess of label to substrate although some substrates had 2 reaction sites and so there would have been only a 4-fold excess of label in these reactions.

*Results*

Various samples which had been previously labelled with phenyl propenyl sulphone, phenyl butenyl sulphone or N-ethyl maleimide (NEM) were analysed by Electrospray Mass Spectrometry (ESMS) and were then subjected to labelling with NEM or PT as appropriate and analysed again by ESMS.

The dipeptide glycine-lysine (GK) was labelled with NEM overnight at RT. Initial ESMS analysis showed that this comprised of 100 % GK $(NEM)_2$, i.e. both amino-groups available on the dipeptide had reacted completely with NEM.

After PT labelling, ESMS analysis showed that the protein was 100 % $GK(NEM)_2(PT)_1$, i.e. the dipeptide had reacted completely with a further molecule of the unhindered sulphone reagent. Previous results (not shown) indicate that the alpha-amino group is resistant to labelling twice even when labelled with an unhindered reagent, so it is assumed that the PT tag has reacted with the epsilon amino group that has already been labelled with one molecule of NEM. This indicates that a peptide labelled with NEM at the epsilon amino group is susceptible to further reaction.

The same dipeptide, GK, was also labelled with phenyl propenyl sulphone (PP). Initial analysis by ESMS showed that this comprised of 92 % $GK(PP)_2$ and 8 % GK(PP), i.e. the majority of the PP had reacted at both free amino groups with the PP tag. After labelling with PT overnight, further analysis by ESMS revealed that the $GK(PP)_2$ species now comprised 72 % $GK(PP)_2$ and 28 % $GK(PP)_2$ (PT). This indicates that PP labelled epsilon amino groups are quite resistant to further reaction with an unhindered alkenyl sulphone.

The dipeptide valine-lysine (VK) was labelled with phenyl isobutenyl sulphone (PBS). This reagent reacts relatively slowly and after 48 hours ESMS analysis showed that this comprised of 43 % no label and 57 % one label. After labelling with PT O/N, a tiny amount of VK(PBS)(PT) was observed. This result indicates that PBS labelled epsilon amino groups are much more resistant to further reaction than phenyl propenyl sulphone labelled amino groups. This is expected as the more bulky hindering group has a greater shielding effect on the tagged amino group.

It is anticipated that epsilon amino groups labelled with hindered alkenyl sulphones such as 1,1,1-trifluoro-3-phenylsulphonylpropene or phenyl hexafluoroisobutenyl sulphone will be even more resistant to further reaction as the trifluoromethyl hindering groups are more hindered than the corresponding methyl groups. Furthermore the electron withdrawing effect of the trifluoromethyl groups will deactivate the adjacent amino group.

Example 5 - Labelling conditions for thiol and epsilon amino group labelling

Since most proteins typically have one or more cysteine residues, which may be cross-linked to form disulphide bridges, and since thiol groups of cysteine are the most reactive side-chains in a polypeptide, it is important that protocols are found that block this functionality as well as any free epsilon amino groups. The hindered Michael reagents used in this invention will react readily with thiols as well as with epsilon amino groups and so both functionalities may be labelled in a single reaction.

Alternatively the thiols may be labelled with a different reagent prior to labelling the epsilon amino groups with the hindered Michael reagents of this invention.

*Capping thiol and epsilon-amino groups with different tags*

In this Example salmon calcitonin (10 nmol, Calbiochem), which has 2 cysteine residues in a disulphide bridge, was dissolved in a denaturing buffer comprising 2 M urea, 0.5 M thiourea in 10 mM sodium carbonate at pH 7.5 in the presence of 0.2 μM tris(carboxyethyl)phosphine (TCEP). TCEP reduces disulphide bridges. The reaction mixture also contained iodoacetamide (20 equivalent per thiol site, 400 nmol) which reacts readily with free thiols. This reaction was left for 90 min. at room temperature. The pH of the buffer was then raised to between 10 and 12 by the addition of sodium hydroxide. Pyridyl propenyl sulphone was then added to the reaction to cap free lysine residues in Salmon Calcitonin. This peptide has 2 lysine residues. The reaction was then desalted (Oasis hydrophilic-lipophilic balance extraction cartridge, Waters) and analysed by MALDI TOF mass spectrometry. The mass spectrum is shown in Figure 5. As can be seen from this mass spectrum a number of different species appear in the mass spectrum corresponding to different labelling products of the peptide. The two different labels give rise to different combinations of incomplete reactions.

*Capping thiol and epsilon amino-groups with the same tag on one peptide*

In this Example 10 nmol of human Calcitonin was dissolved in a denaturing buffer comprising 2 M urea, 0.5 M thiourea in 10 mM sodium carbonate at pH 7.5 in the presence of 0.2 μM tris(carboxyethyl)phosphine (TCEP). TCEP reduces disulphide bridges. This reaction was left for 30 minutes to allow complete reduction of all disulphide bridges to take place. After the reduction reaction 40 equivalents of pyridyl propenyl sulphone per reaction site, which were assumed only to comprise epsilon amino groups and thiol groups, was added to the reaction mixture. This reaction was left for 90 min. at room temperature at pH 8. The pH of the buffer was then raised to between 11-12 by the addition of sodium hydroxide. The reaction mixture was left at the higher pH for 4 hours at room temperature to cap free lysine residues in the peptides. Unreacted tag was quenched with an excess of lysine. The reaction was then desalted (Oasis hydrophilic-lipophilic balance extraction cartridge, Waters) and analysed by MALDI TOF mass spectrometry. The mass spectrum is shown in Figure 6. As can be seen from this mass spectrum the number of different species appearing in the mass spectrum corresponding to different labelling products of each peptide is much smaller than for the protocol using two different tags for thiols and epsilon amino groups.

*Capping thiol and epsilon-amino groups with the same tag on a mixture of peptides*

In this Example a mixture of peptides (10 nmol of each) comprising beta-melanocyte stimulating hormone (β-MSH), alpha-melanocyte stimulating hormone (α-MSH), Salmon Calcitonin and residues 1 to 24 of adrenocorticotropic hormone (ACTH (1-24)) (all available from Sigma-Aldrich, Dorset, UK) were dissolved in a denaturing buffer comprising 2 M urea, 0.5 M thiourea in 10 mM sodium borate at pH 7.5 in the presence of 0.2 μM TCEP. This reaction was left for 30 minutes to allow complete reduction of all disulphide bridges to take place. After the reduction reaction 40 equivalents of pyridyl propenyl sulphone per reaction site, which were assumed only to comprise epsilon amino groups and thiol groups, was added to the reaction mixture. This reaction was left for 90 min. at room temperature at

pH 8. The pH of the buffer was then raised to between 11-12 by the addition of sodium hydroxide. The reaction mixture was left at the higher pH for 4 hours at room temperature to cap free lysine residues in the peptides. Unreacted tag was quenched with an excess of lysine. The reaction was then desalted (Oasis hydrophilic-lipophilic balance extraction cartridge, Waters) and analysed by MALDI TOF mass spectrometry. The mass spectrum is shown in Figure 7. As can be seen from this mass spectrum the number of different species appearing in the mass spectrum corresponding to different labelling products of each peptide is much smaller than for the protocol using two different tags for thiols and epsilon amino groups.

*Capping of unblocked alpha-amino groups*

Following the capping of the mixture of peptides above, the unblocked alpha-amino groups were blocked with acetic acid N-hydroxysuccinimide ester. The thiol and epsilon amino capped peptides were exposed to 40 equivalents of the active ester reagent per alpha amino group in the same sodium borate buffer used previously at pH 11 for 2 hours at room temperature. The MALDI TOF mass spectrum of the products of this reaction is shown in Figure 8. As can be seen from this Figure, only one acetyl group reacts with each of the peptides that are expected to react, i.e. all of the four peptides except α-MSH. This means that the capped epsilon amino groups are resistant to reaction with the active ester reagent.

Example 6 - Isolation of N-terminal peptides from a mixture of small polypeptides using enzymatic cleavage

In this Example a mixture of peptides (10 nmol of each) comprising beta-melanocyte stimulating hormone (β-MSH), alpha-melanocyte stimulating hormone (α-MSH), Salmon Calcitonin, Human Calcitonin and residues 1 to 24 of adrenocorticotropic hormone (ACTH (1-24)) (all available from Sigma-Aldrich, Dorset, UK) were capped on thiols and epsilon amino groups with pyridyl propenyl sulphone using the protocol of the previous Example. Similarly the available alpha-amino groups of these peptides were capped with acetic acid N-hydroxysuccinimide ester as described in the previous Example. The unreacted tags were quenched with an excess of cysteine. The capped peptides were then treated with trypsin at a concentration of 1/50 (wt. peptides/wt. enzyme) in 150 mM sodium borate at pH 8 which now cleaved only at the arginine residues in these peptides exposing new alpha amino groups in the non-N-terminal cleaved peptides.

The cleavage mixture was then treated with N-hydroxysuccinimidyl biotin in DMSO. 50 equivalents of biotin reagent per available amino group were used. The reaction mixture was then passed through a neutravidin affinity column (from Pierce Ltd) in a PBS buffer at pH 7.5 (1 ml of affinity reagent used with 12-15 μM of avidin per ml of resin). The reaction mixture was left for 30 min. in the affinity column so that the biotin moiety binds to the streptavidin.

The peptides left in the solution phase, which should be the N-terminal peptides were desalted (Oasis hydrophilic-lipophilic balance extraction cartridge, Waters) and analysed by MALDI TOF mass spectrometry. The spectra are shown in Figures 9 to 11. Figure 9 shows the region of the spectrum with the expected peaks for the N-terminal peptides of α-MSH, β-MSH and ACTH (1-24). Different species corresponding to different numbers of pyridyl propenyl sulphone mass tags are found for each peptide, some labelling of histidine residues may be taking place. Figure 10 shows the region of the spectrum with the expected peaks for the N-terminal peptides of Calcitonin S and Calcitonin H. The expected peaks and some extra labelling peaks are found. Finally, Figure 11 shows the low mass region of the spectrum where any contaminating C-terminal peptides would be found if they were present. No large peaks corresponding to the C-terminal peptides are observed. Some very low intensity peaks can be seen if the relevant regions of the spectrum are enlarged, which may indicate a very low level of contamination by C-terminal peptides (data not shown).

These data confirm the efficacy of the present invention in identifying N-terminal peptides for characterisation of protein and polypeptide samples.

**Claims**

**1.** A method for characterising a polypeptide or a population of polypeptides, which method comprises the steps of:

(a) contacting a sample comprising one or more polypeptides with a lysine reactive agent to cap ε-amino groups;
(b) optionally reacting the sample of polypeptides with an amine reactive reagent to block α-amino groups;
(c) digesting the sample of polypeptides with a sequence specific cleavage reagent to produce peptide fragments;
(d) optionally deactivating the cleavage reagent;

(e) removing those peptides having uncapped or unblocked amino groups; and
(f) recovering the N-terminal peptides.

**2.** A method according to claim 1 wherein the polypeptide or polypeptides comprise one or more N-terminal amine groups that are naturally unblocked, which method comprises reacting the sample of polypeptides with an amine reactive reagent to block α-amino groups according to step (b).

**3.** A method according to claim 1 wherein the polypeptide or polypeptides comprise N-terminal amine groups that are naturally blocked, which method does not comprise reacting the sample of polypeptides with an amine reactive reagent to block α-amino groups according to step (b).

**4.** A method according to any preceding claim, wherein non-N-terminal peptides and/or naturally unblocked N-terminal peptides are removed by capturing them on a solid phase and N-terminal peptides are recovered in solution.

**5.** A method for characterising a polypeptide or a population of polypeptides, which method comprises the steps of:

(a) contacting a sample comprising one or more polypeptides with a lysine reactive agent to cap ε-amino groups;
(b) contacting the resultant capped polypeptides with an amine reactive agent which reacts with the unblocked α-amino groups at the N-termini of the polypeptides;
(c) digesting the sample of polypeptides with a sequence specific cleavage agent to produce peptide fragments;
(d) optionally deactivating the cleavage reagent; and
(e) recovering N-terminal peptides that have reacted with the amine reactive agent.

**6.** A method according to claim 5, wherein N-terminal peptides are recovered by capturing them on a solid phase and non-N-terminal peptides are removed in solution.

**7.** A method according to claim 6, wherein the amine reactive agent or the lysine reactive agent is attached to a solid phase.

**8.** A method according to claim 6 or claim 7, wherein the amine reactive agent comprises biotin and the solid phase is an avidinated solid phase.

**9.** A method according to any of claims 6-8, wherein two or more samples are reacted with differently labelled amine reactive agents, and subsequently the samples are pooled and analysed simultaneously.

**10.** A method according to claim 9, wherein at least one of the amine reactive agents is labelled with deuterium and the samples are analysed by mass spectrometry.

**11.** A method according to any preceding claim, wherein only one molecule of the lysine reactive agent reacts with each ε-amine group available in the peptides or polypeptides.

**12.** A method according to any preceding claim, wherein the lysine reactive agent comprises a hindered Michael reagent.

**13.** A method according to any preceding claim, wherein the hindered Michael agent comprises a compound having the following structure:

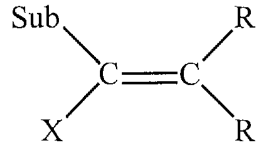

wherein X is an electron withdrawing group that is capable of stabilising a negative charge; the R groups independently comprise a hydrogen, a halogen, an alkyl, an aryl, or an aromatic group with the proviso that at least one of the R groups comprises a sterically hindering group; and the group Sub comprises a hydrogen, a halogen, a hydrocarbon group or an electron withdrawing group.

**14.** A method according to claim 13, wherein one R comprises a methyl or phenyl group.

**15.** A method according to claim 13 or claim 14 wherein at least one R comprises an electron withdrawing group.

**16.** A method according to any of claims 13-15, wherein at least one R comprises a cyclic or heterocylic aromatic ring or fused ring.

**17.** A method according to any of claims 13-16, wherein X comprises an $-SO_2R^1$ group, wherein $R^1$ comprises an alkyl group or an aryl group, including aromatic groups cyclic groups, fused cyclic groups, and heterocyclic groups.

**18.** A method according to claim 17, wherein $R^1$ comprises an electron withdrawing group.

**19.** A method according to claim 17 or claim 18, wherein the ring comprises a phenyl, pyridyl, naphthyl quinolyl, pyrazine, pyrimidine or triazine ring structure.

**20.** A method according to any of claims 13-19 wherein the X group is substituted with an electron withdrawing group.

**21.** A method according to claim 20, wherein the electron withdrawing group is selected from halogens, such as fluorine chlorine, bromine or iodine, and nitro and nitrile groups.

**22.** A method according to any of claims 13-21, wherein the X group comprises a structure capable of promoting water solubility.

**23.** A method according to any one of the preceding claims, wherein the cleavage agent comprises a peptidase.

**24.** A method according to claim 23, wherein the peptidase comprises trypsin, Lys-C, Arg-C, Cyanogen Bromide or BNPS-Skatole.

**25.** A method according to any one of the preceding claims, wherein the sample of step (a) comprises a sub-cellular fraction.

**26.** A method according to any one of the preceding claims, which further comprises preparing the sample of step (a) by liquid chromatography.

**27.** A method for assaying for one or more specific polypeptides in a test sample, which comprises performing a method according to any one of claims 1 to 26, wherein the sequence of the specific polypeptide is determined by assaying the resulting N-termini for a predetermined N-terminal sequence of amino acid residues.

**28.** A method of characterising one or more mixtures of polypeptides, which method comprises the following steps:

(a) recovering one or more N-terminal peptides from the mixtures by employing one or more of the methods as defined in any of claims 1-26;
(b) detecting the peptides by mass spectrometry.

**29.** A method for determining the 'expression profile' of a sample, which method comprises characterising one or more mixtures of polypeptides according to a method as defined in claim 25.

**30.** A method according to claim 28 or claim 29, which method comprises determining the identity of each of the peptides detected by mass spectrometry.

**31.** A method according to any of claims 28-30, which method comprises identifying the quantity of each of the peptides detected by mass spectrometry.

**32.** A method for characterising a polypeptide or a population of polypeptides, which method comprises contacting a

sample comprising one or more polypeptides with a lysine reactive agent to attach the agent to ε-amino groups, wherein the lysine reactive agent comprises a hindered Michael reagent.

**33.** A method according to claim 32, wherein the hindered Michael agent is a compound as defined in any of claims 13-22.

**34.** A compound having the following structure:

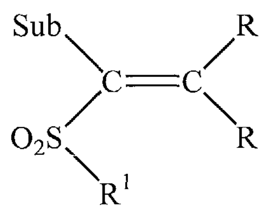

wherein $R^1$ comprises a pyridyl, quinolyl, pyrazine, pyrimidine or triazine ring structure and the R groups independently comprise a hydrogen, a halogen, or an alkyl or aryl group with the proviso that at least one of the R groups comprises a sterically hindering group; and the group Sub comprises a hydrogen, a halogen, a hydrocarbon group or an electron withdrawing group.

**35.** A compound according to claim 34, wherein at least one R group comprises a methyl or phenyl group.

**36.** A compound according to claim 34 or claim 35, wherein at least one R group comprises an electron-withdrawing group.

**37.** A compound according to claim 36 wherein at least one R group comprises a halogen atom or a halogenated alkyl group, or a phenyl ring with one or more electron withdrawing substituents.

**38.** A kit for characterising a polypeptide or a population of polypeptides, which kit comprises:

(a) a lysine reactive agent for capping ε-amino groups;
(b) a means for recovering or isolating N-terminal peptides;
(c) optionally an amine reactive reagent for blocking α-amino groups;
(d) optionally a sequence specific cleavage reagent for producing peptide fragments.

**39.** A kit according to claim 38, wherein the lysine reactive agent comprises a compound having the following structure:

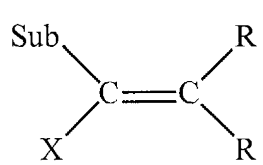

wherein X is an electron withdrawing group that is capable of stabilising a negative charge; the R groups independently comprise a hydrogen, a halogen, an alkyl, an aryl, or an aromatic group with the proviso that at least one of the R groups comprises a sterically hindering group; and the group Sub comprises a hydrogen, a halogen, a hydrocarbon group or an electron withdrawing group.

**40.** A kit according to claim 39, wherein the lysine reactive agent comprises a compound as defined in any of claims 34-37.

**41.** A kit according to claim 39 or claim 40, wherein the a means for recovering or isolating N-terminal peptides comprises a solid phase adapted for capturing peptides comprising free α-amino groups.

**42.** Use of a compound having the following structure for protecting ε-amino groups in peptides and polypeptides:

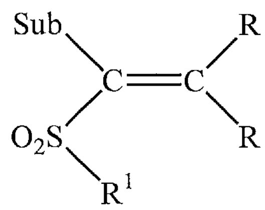

wherein $R^1$ comprises an alkyl group or an aryl group, including aromatic groups cyclic groups, fused cyclic groups, and heterocyclic groups, and the R groups independently comprise a hydrogen, a halogen, or an alkyl or aryl group with the proviso that at least one of the R groups comprises a sterically hindering group; and the group Sub comprises a hydrogen, a halogen, a hydrocarbon group or an electron withdrawing group.

**43.** Use according to claim 42, wherein $R^1$ comprises a pyridyl, quinolyl, pyrazine, pyrimidine or triazine ring structure.

**44.** Use according to claim 42 or claim 43, wherein at least one R group comprises a methyl or phenyl group.

**45.** Use according to any of claims 42-44, wherein at least one R group comprises an electron-withdrawing group.

**46.** Use according to claim 45 wherein at least one R group comprises a halogen atom or a halogenated alkyl group, or a phenyl ring with one or more electron withdrawing substituents.

**47.** Use according to any of claims 42-46, wherein the protection is against further reaction of the ε-amino groups with Edman agents, capture agents and agents which are capable of reacting with α-amino groups.

**48.** Use according to claim 47, wherein the Edman agent comprises an isothiocyanate or an isocyanate, the capture agent comprises N-hydroxysuccinimidyl biotin and the agent which is capable of reacting with α-amino groups comprises acetic acid N-hydroxysuccinimide ester.

FIG. 1

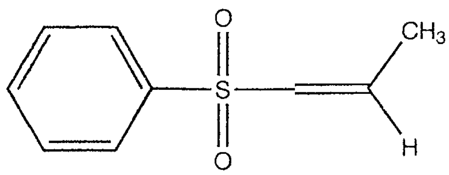

Phenyl-1-propenyl sulphone

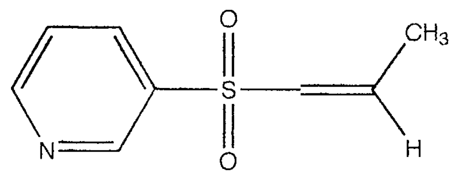

Pyridyl-1-propenyl sulphone

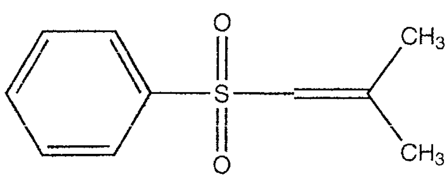

Phenyl-1-isobutenyl sulphone

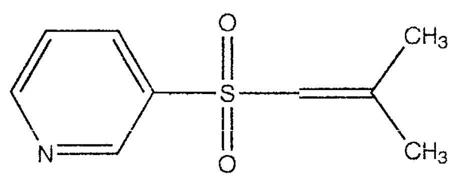

Pyridyl-1-isobutenyl sulphone

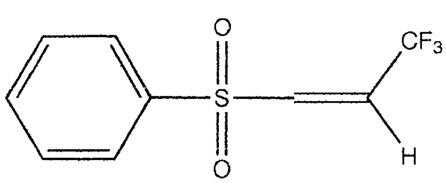

3,3,3-trifluoro-1-(phenylsulphonyl)-1-propene or phenyl trifluoropropenyl sulphone

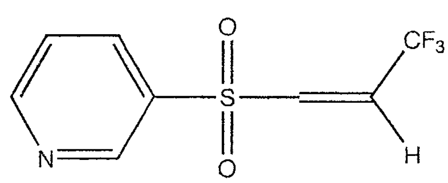

3,3,3-trifluoro-1-(pyridylsulphonyl)-1-propene or pyridyl trifluoropropenyl sulphone

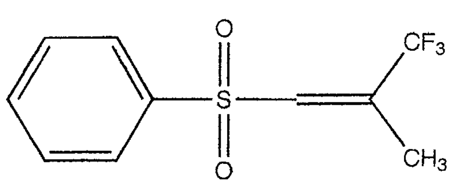

2-trifluoromethyl-2-methyl-phenyl-3-vinylsulphone or phenyl trifluoroisobutenyl sulphone

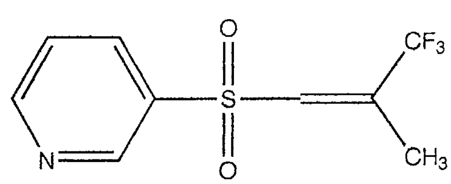

2-trifluoromethyl-2-methyl-pyridine-3-vinylsulphone or pyridyl trifluoroisobutenyl sulphone

FIG. 2a

**Isolation of naturally blocked and unblocked N-terminal Peptides:**

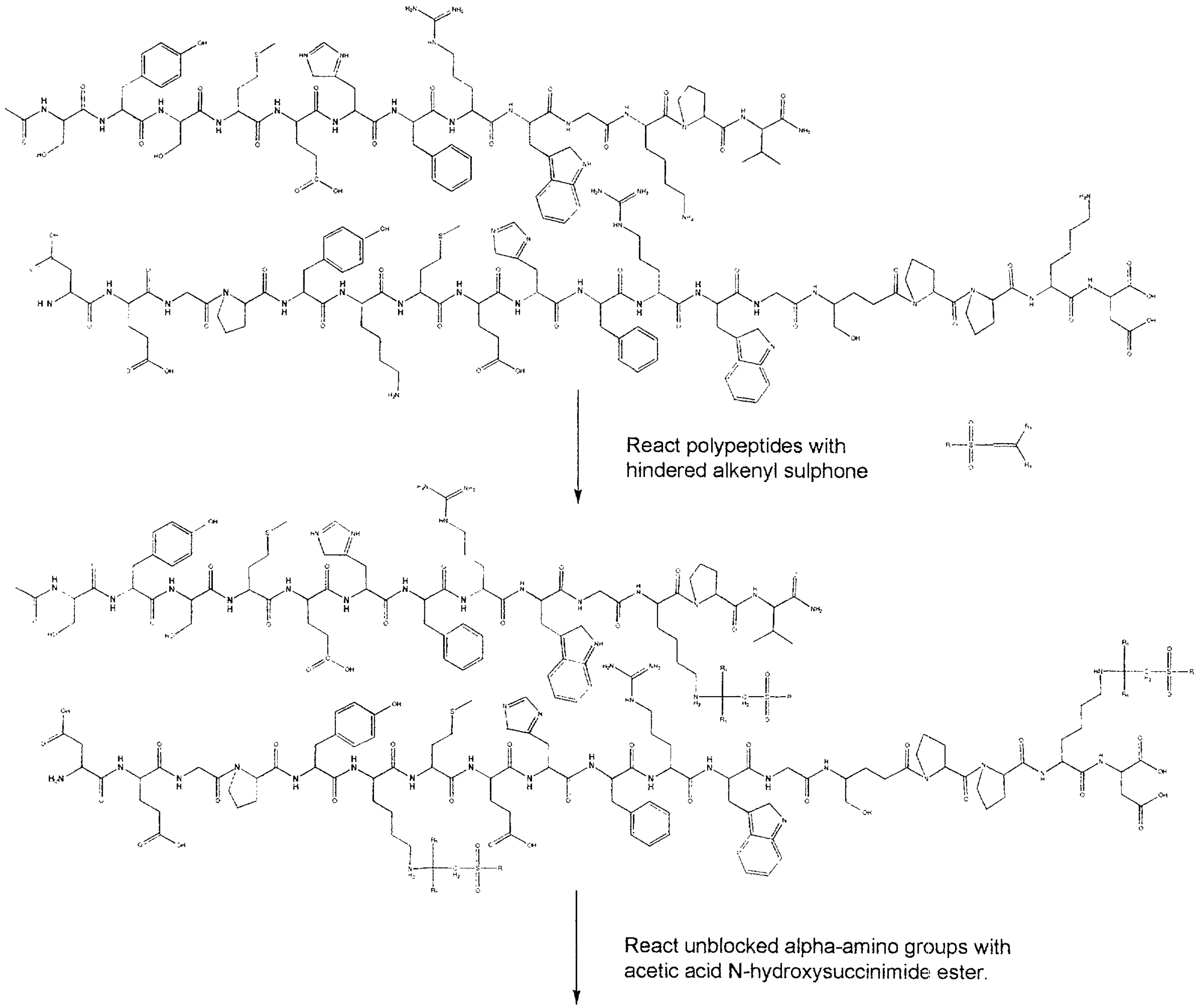

FIG. 2b

**Isolation of naturally blocked and unblocked N-terminal Peptides, continued from figure 2(a):**

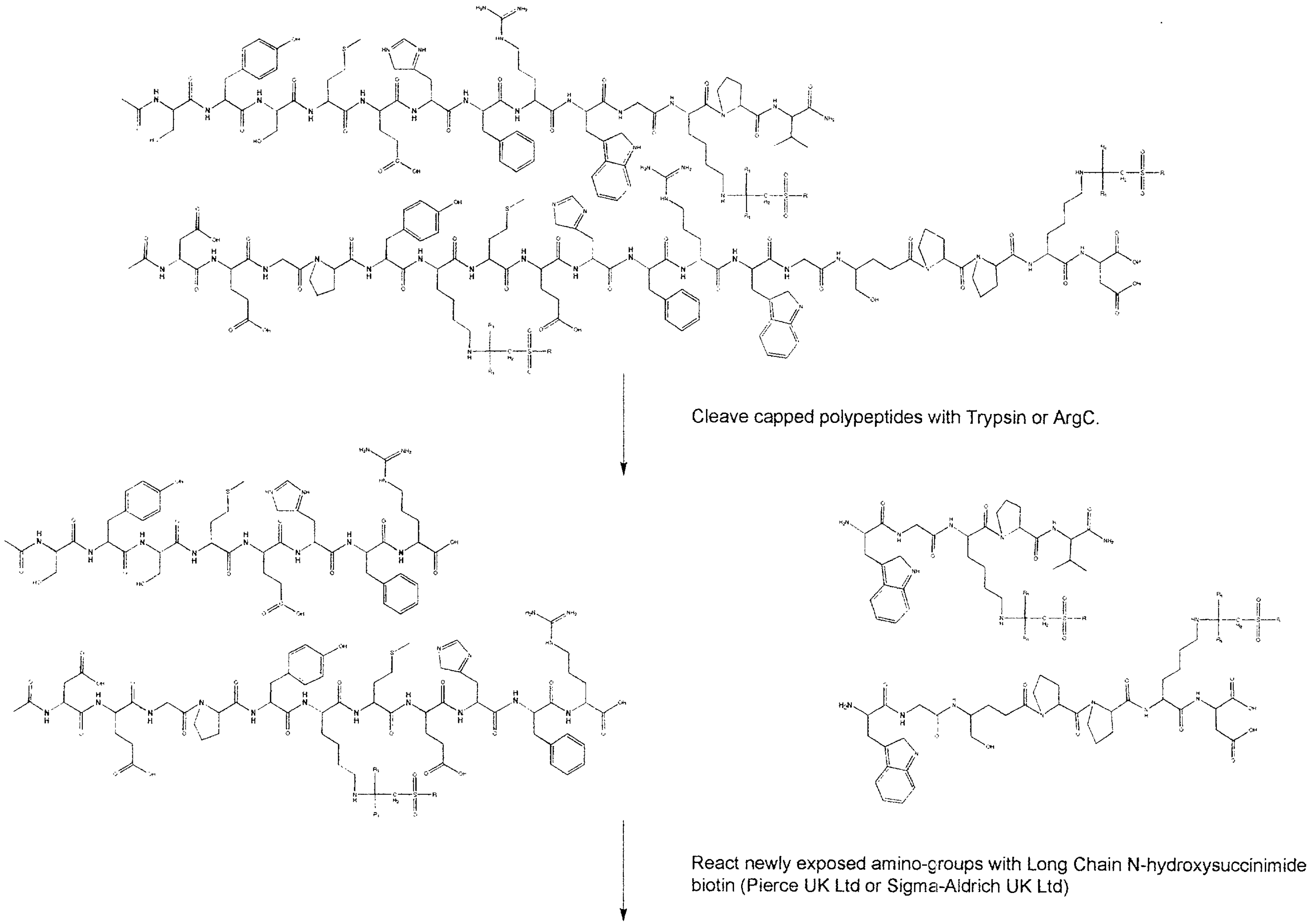

FIG. 2c

**Isolation of naturally blocked and unblocked N-terminal Peptides, continued from figure 2(b):**

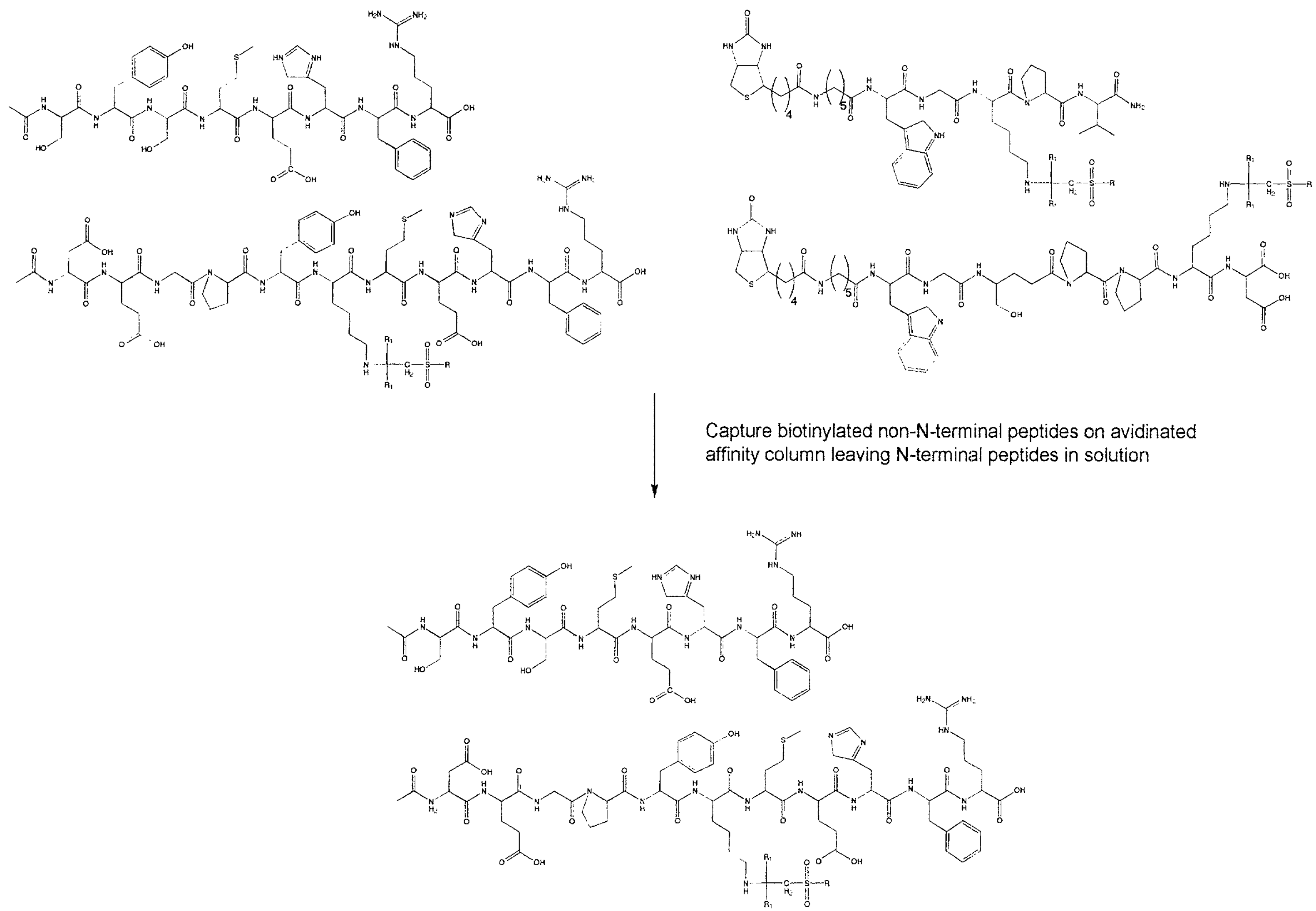

FIG. 3a

**Isolation of naturally blocked N-terminal Peptides:**

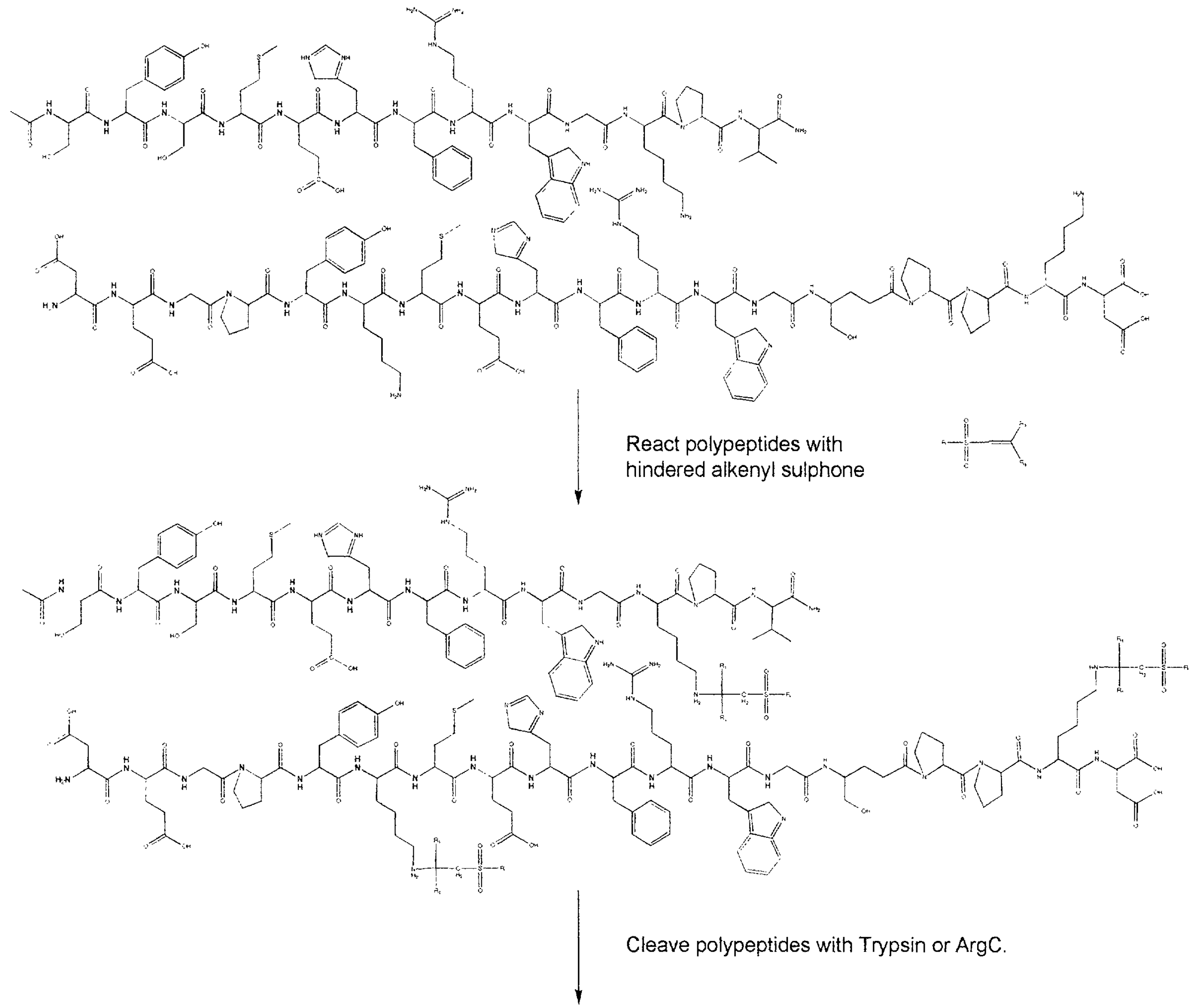

FIG. 3b

**Isolation of naturally blocked N-terminal Peptides, continued from figure 3(a):**

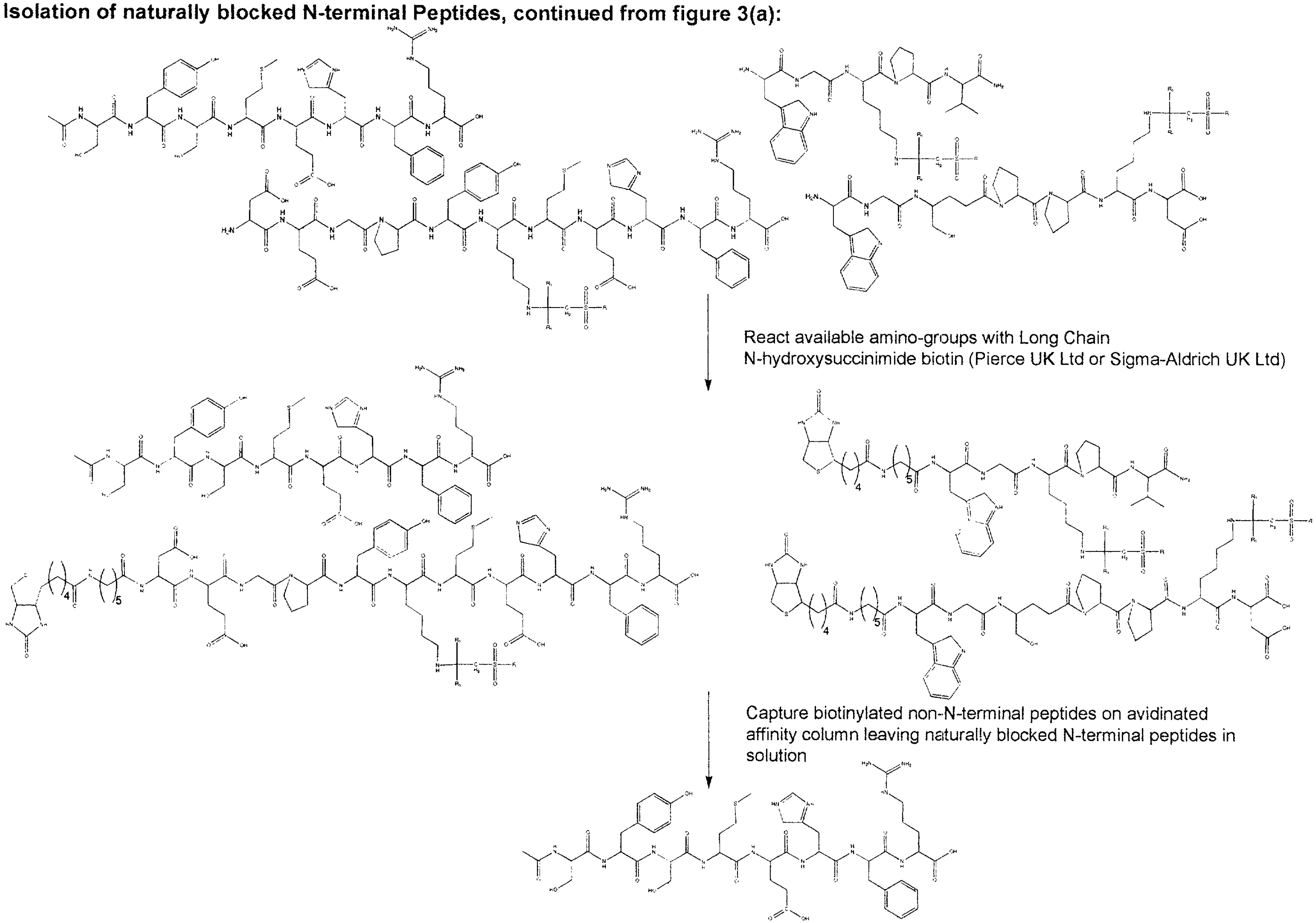

FIG. 4a

**Isolation of naturally unblocked N-terminal Peptides:**

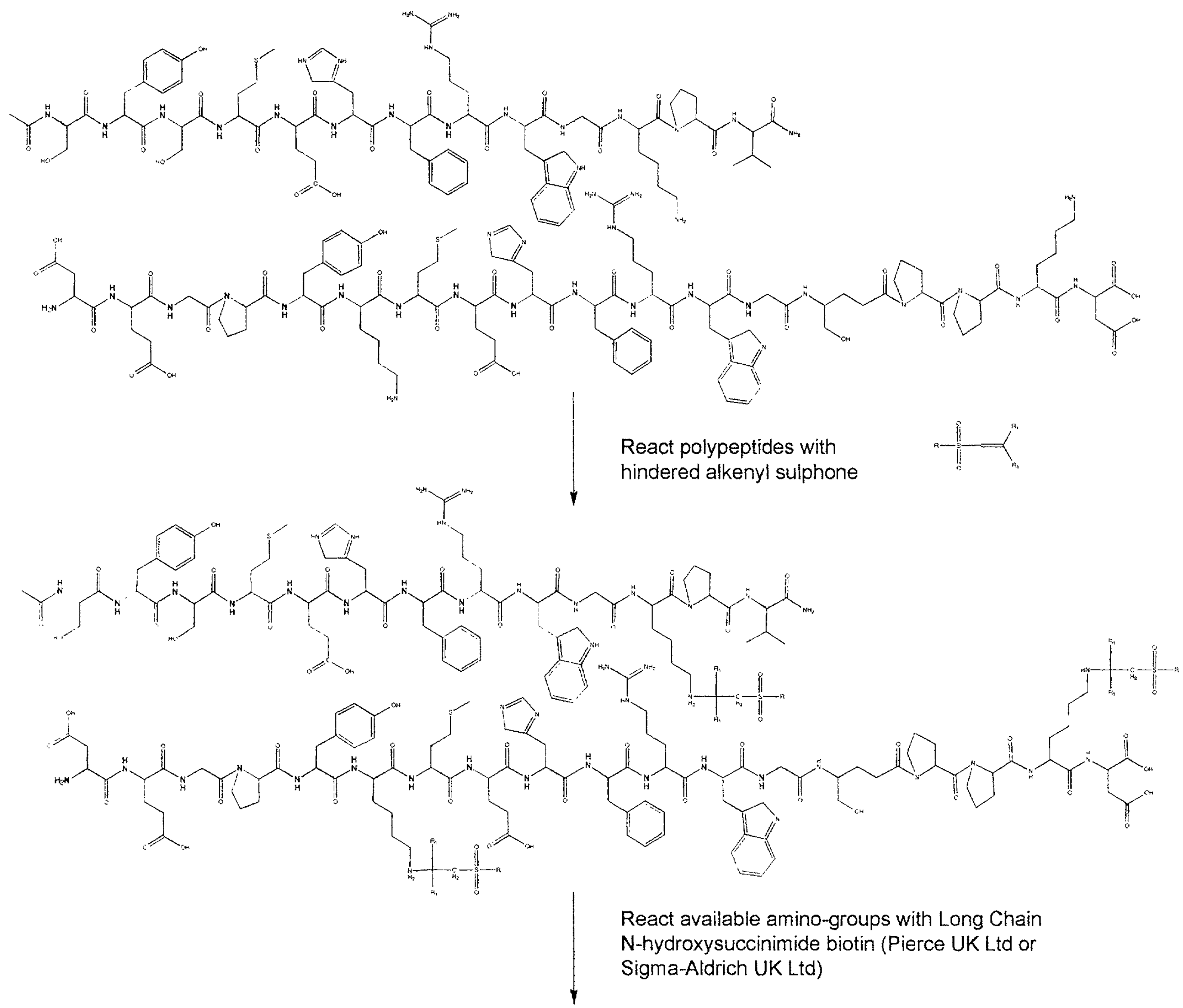

FIG. 4b

**Isolation of naturally unblocked N-terminal Peptides, continued from figure 4(a):**

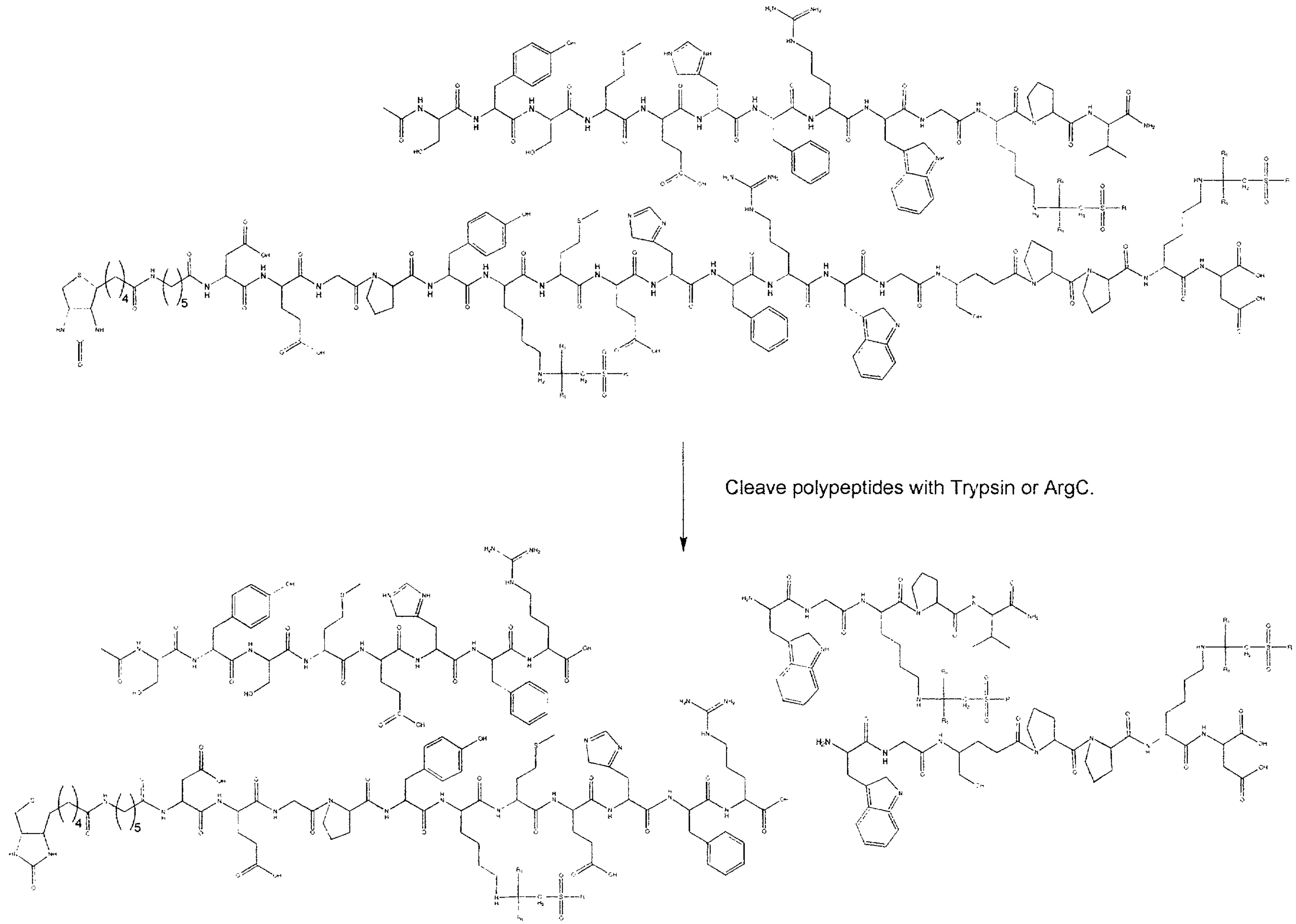

FIG. 4c

**Isolation of naturally unblocked N-terminal Peptides, continued from figure 4(b):**

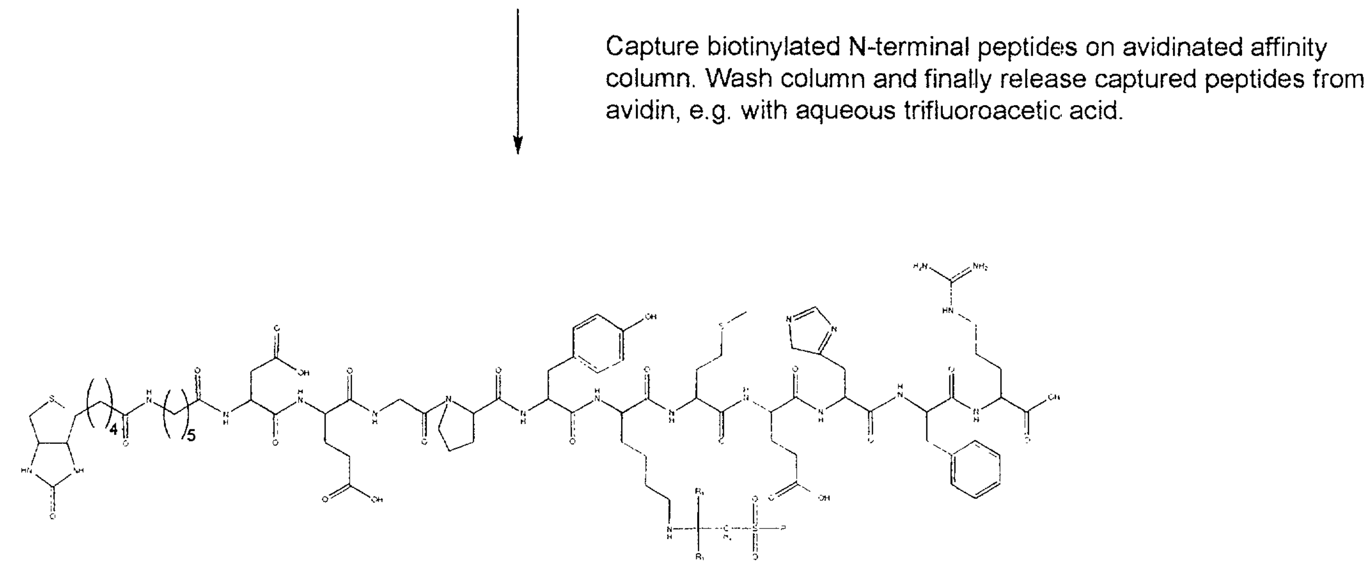

FIG. 5

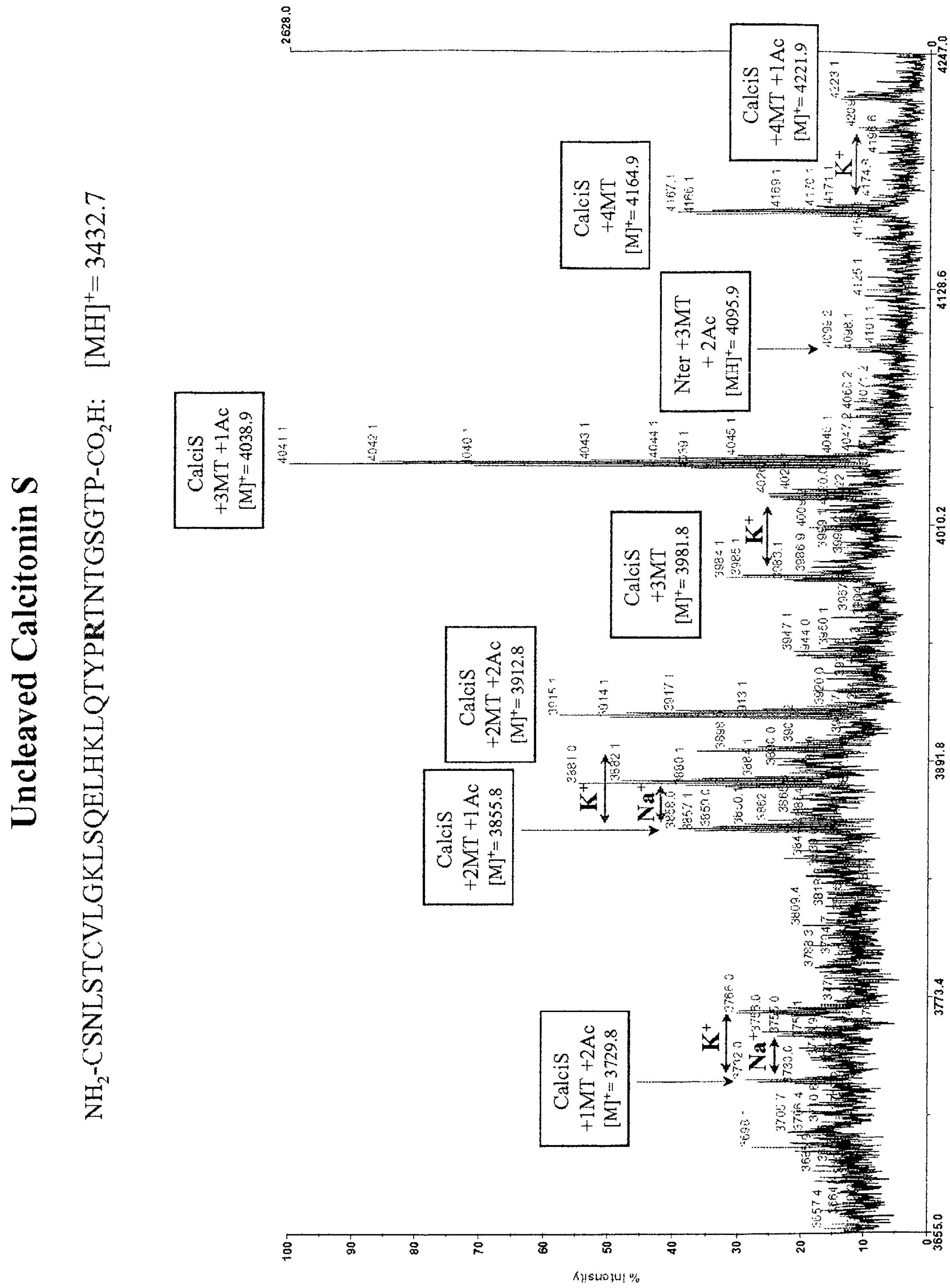
Uncleaved Calcitonin S
$NH_2$-CSNLSTCVLGKLSQELHKLQTYPRTNTGSGTP-$CO_2H$: $[MH]^+$= 3432.7
CalciS +1MT +2Ac $[M]^+$= 3729.8
CalciS +2MT +1Ac $[M]^+$= 3855.8
CalciS +2MT +2Ac $[M]^+$= 3912.8
CalciS +3MT $[M]^+$= 3981.8
CalciS +3MT +1Ac $[M]^+$= 4038.9
Nter +3MT + 2Ac $[MH]^+$= 4095.9
CalciS +4MT $[M]^+$= 4164.9
CalciS +4MT +1Ac $[M]^+$= 4221.9
$K^+$
$Na^+$
% Intensity
Mass (m/z)
3655.0
3773.4
3891.8
4010.2
4128.6
4247.0
2628.0

FIG. 6

Human Calcitonin + 3 Mass Tags

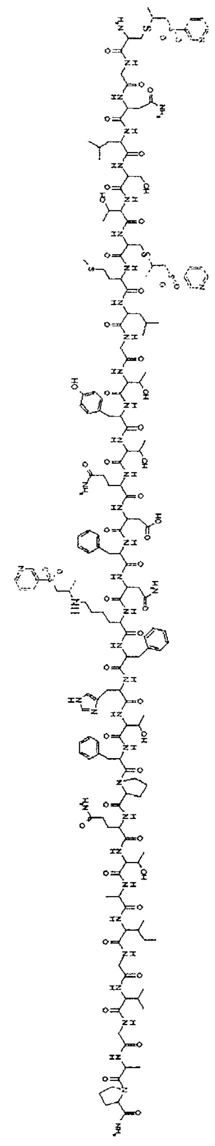

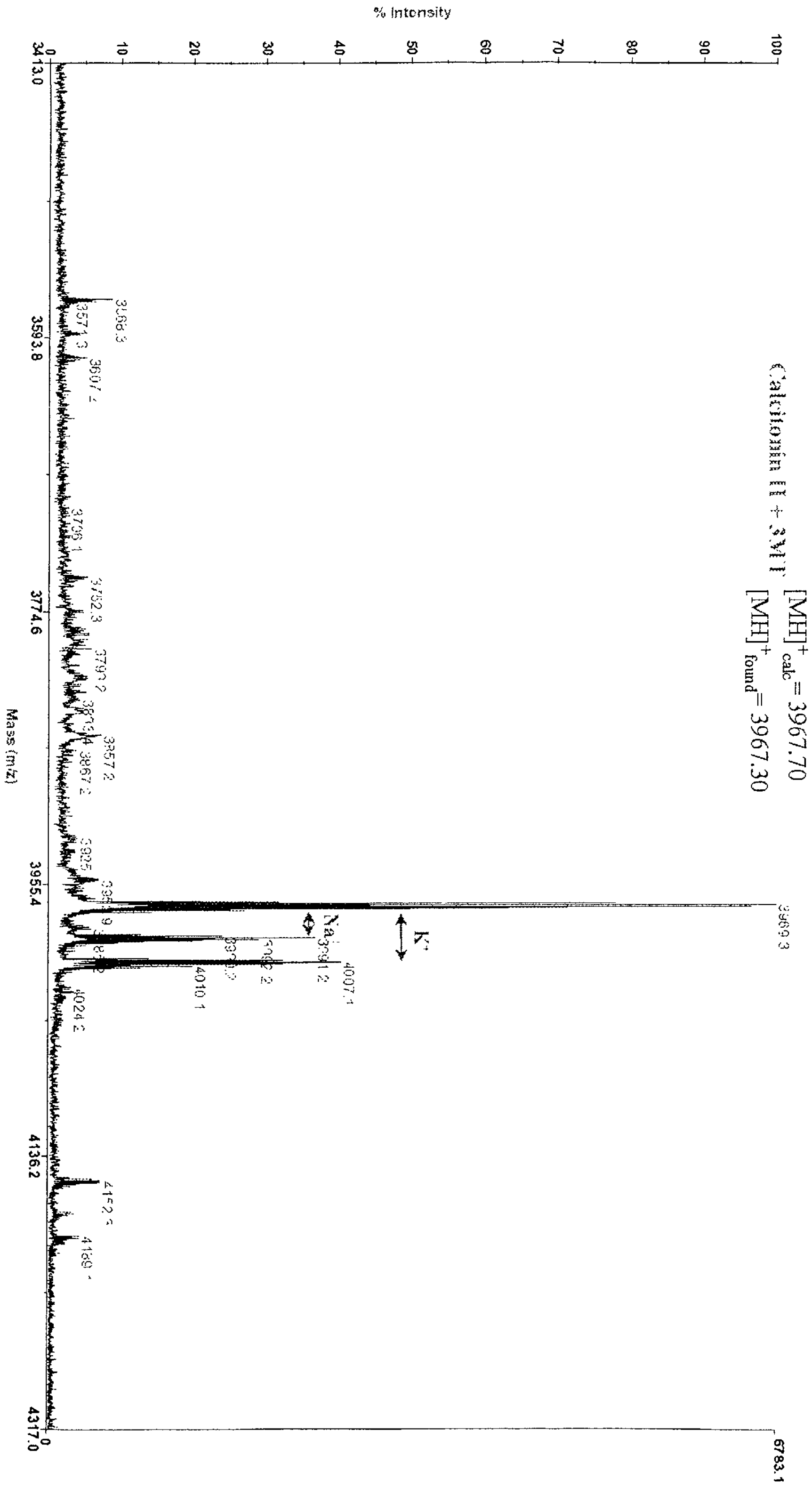

FIG. 7

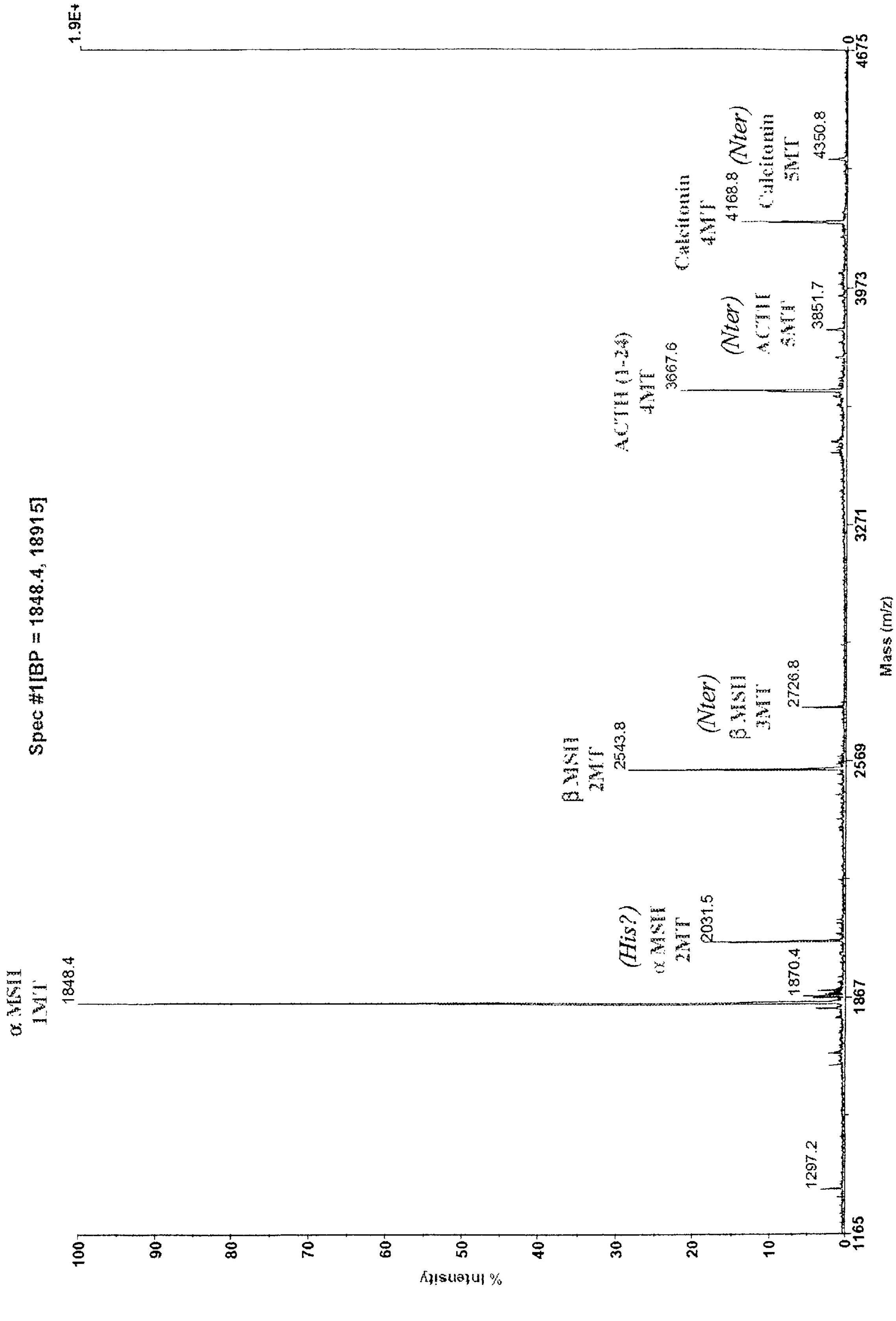

Spec #1[BP = 1848.4, 18915]
1.9E+4
% Intensity
Mass (m/z)
1165
1867
2569
3271
3973
4675
1297.2
1848.4
α MSH
1MT
1870.4
2031.5
(His?)
α MSH
2MT
2543.8
β MSH
2MT
2726.8
(Nter)
β MSH
3MT
3667.6
ACTH (1-24)
4MT
3851.7
(Nter)
ACTH
5MT
4168.8
Calcitonin
4MT
4350.8
(Nter)
Calcitonin
5MT

FIG. 8

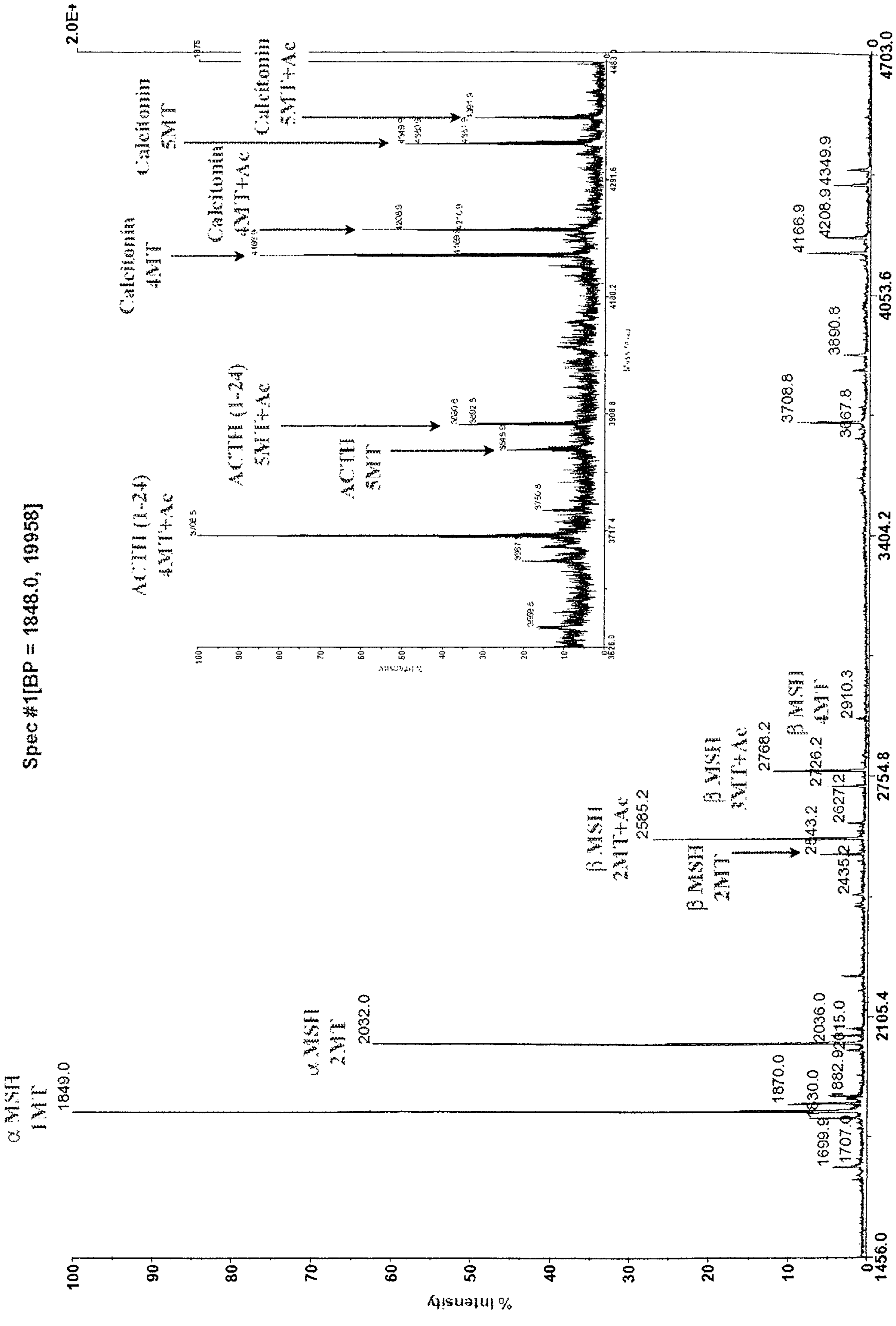
Spec #1[BP = 1848.0, 19958]
% Intensity
Mass (m/z)
α MSH 1MT
α MSH 2MT
β MSH 2MT
β MSH 2MT+Ac
β MSH 3MT+Ac
β MSH 4MT
ACTH (1-24) 4MT+Ac
ACTH (1-24) 5MT+Ac
ACTH 5MT
Calcitonin 4MT
Calcitonin 4MT+Ac
Calcitonin 5MT
Calcitonin 5MT+Ac
1849.0
2032.0
2585.2
2768.2
2910.3
3708.8
3890.8
4166.9
4208.9
4349.9
1456.0
2105.4
2754.8
3404.2
4053.6
4703.0
2.0E+

FIG. 9

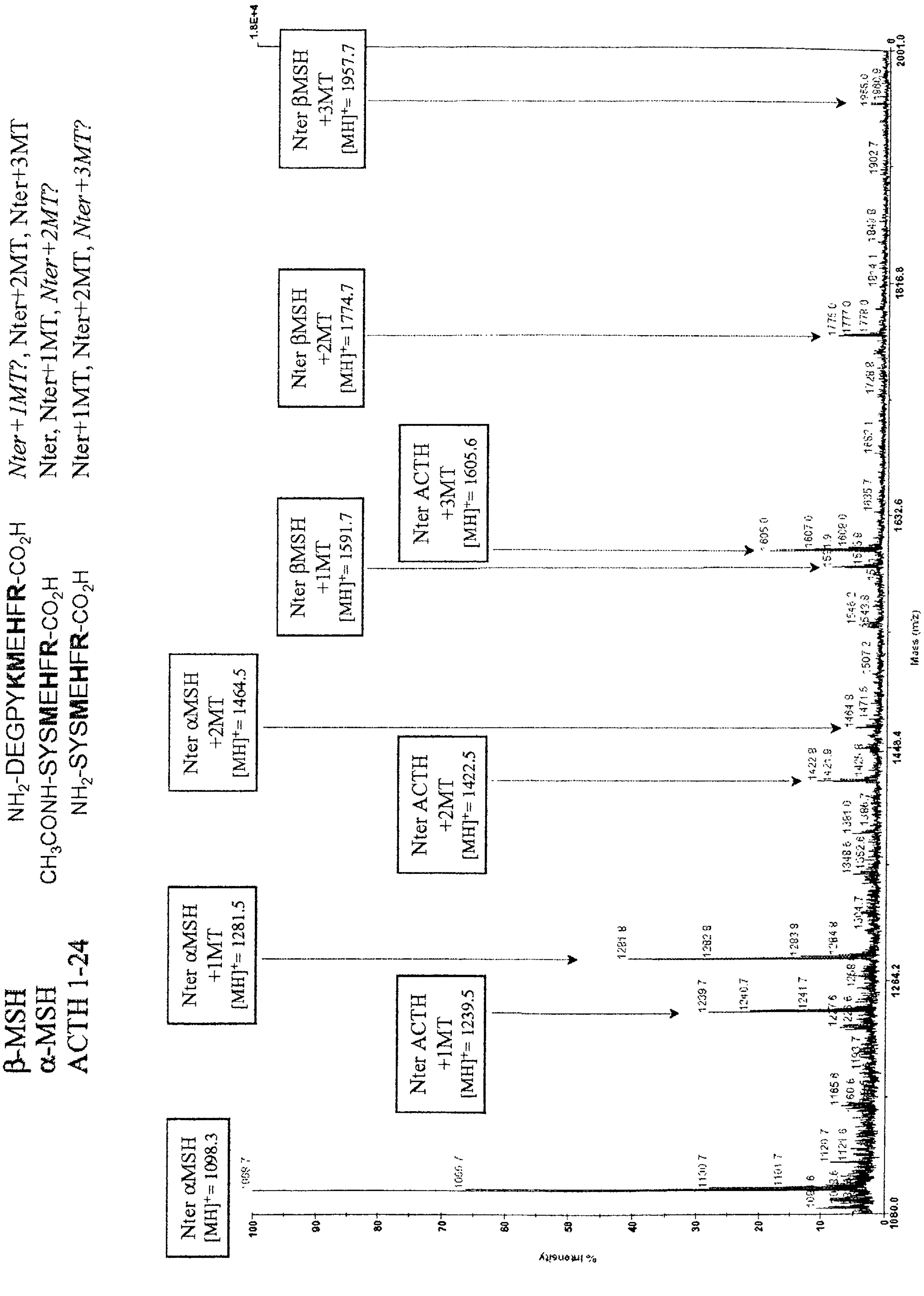

β-MSH
α-MSH
ACTH 1-24
NH2-DEGPYKMEHFR-CO2H
CH3CONH-SYSMEHFR-CO2H
NH2-SYSMEHFR-CO2H
Nter+1MT?, Nter+2MT, Nter+3MT
Nter, Nter+1MT, Nter+2MT?
Nter+1MT, Nter+2MT, Nter+3MT?
Nter αMSH [MH]+= 1098.3
Nter ACTH +1MT [MH]+= 1239.5
Nter αMSH +1MT [MH]+= 1281.5
Nter ACTH +2MT [MH]+= 1422.5
Nter αMSH +2MT [MH]+= 1464.5
Nter βMSH +1MT [MH]+= 1591.7
Nter ACTH +3MT [MH]+= 1605.6
Nter βMSH +2MT [MH]+= 1774.7
Nter βMSH +3MT [MH]+= 1957.7
% Intensity
Mass (m/z)
1.8E+4
1080.0
1264.2
1448.4
1632.6
1816.8
2001.0

FIG. 10

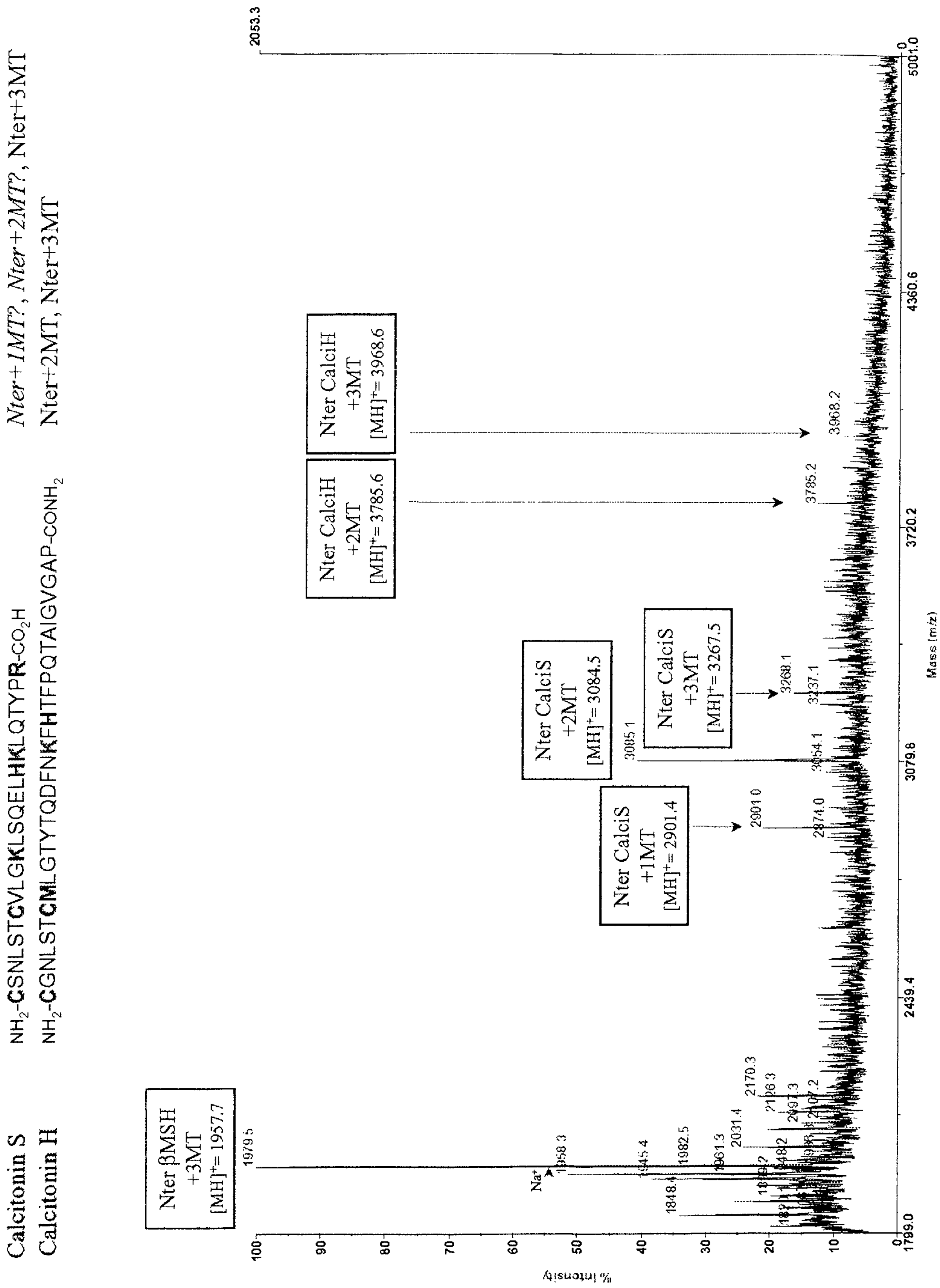
Calcitonin S NH2-CSNLSTCVLGKLSQELHKLQTYPR-CO2H Nter+1MT?, Nter+2MT?, Nter+3MT
Calcitonin H NH2-CGNLSTCMLGTYTQDFNKFHTFPQTAIGVGAP-CONH2 Nter+2MT, Nter+3MT
Nter βMSH +3MT [MH]+= 1957.7
Nter CalciS +1MT [MH]+= 2901.4
Nter CalciS +2MT [MH]+= 3084.5
Nter CalciS +3MT [MH]+= 3267.5
Nter CalciH +2MT [MH]+= 3785.6
Nter CalciH +3MT [MH]+= 3968.6
Na+
1979.5
2053.3
2901.0
3085.1
3268.1
3785.2
3968.2
% Intensity
Mass (m/z)
1799.0
2439.4
3079.8
3720.2
4360.6
5001.0

FIG. 11

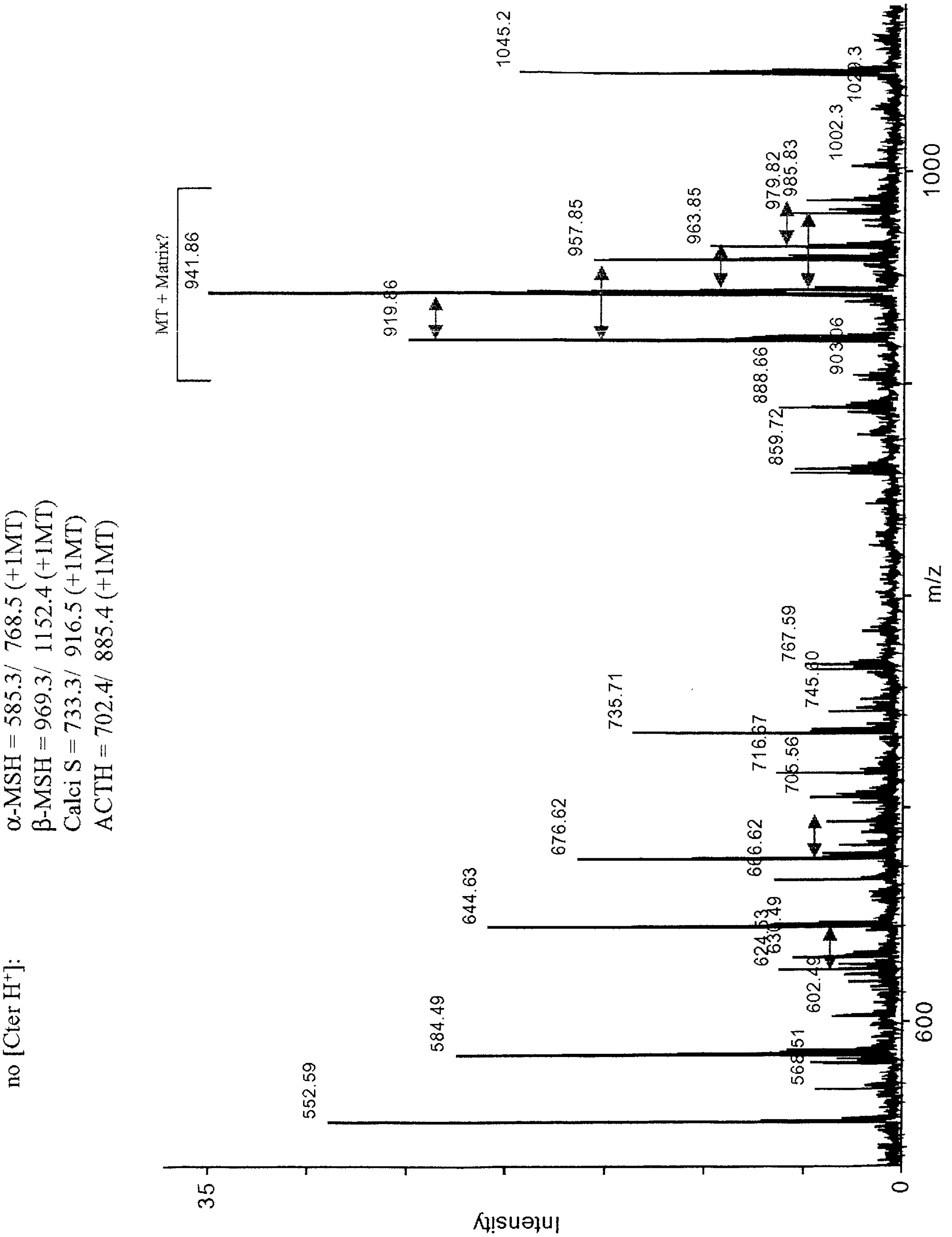
no [Cter H+]:
α-MSH = 585.3/ 768.5 (+1MT)
β-MSH = 969.3/ 1152.4 (+1MT)
Calci S = 733.3/ 916.5 (+1MT)
ACTH = 702.4/ 885.4 (+1MT)
MT + Matrix?
552.59
584.49
644.63
676.62
735.71
919.86
941.86
957.85
963.85
979.82
985.83
1002.3
1045.2
888.66
859.72
767.59
716.67
705.56
602.49
Intensity
35
0
600
1000
m/z

FIG. 12

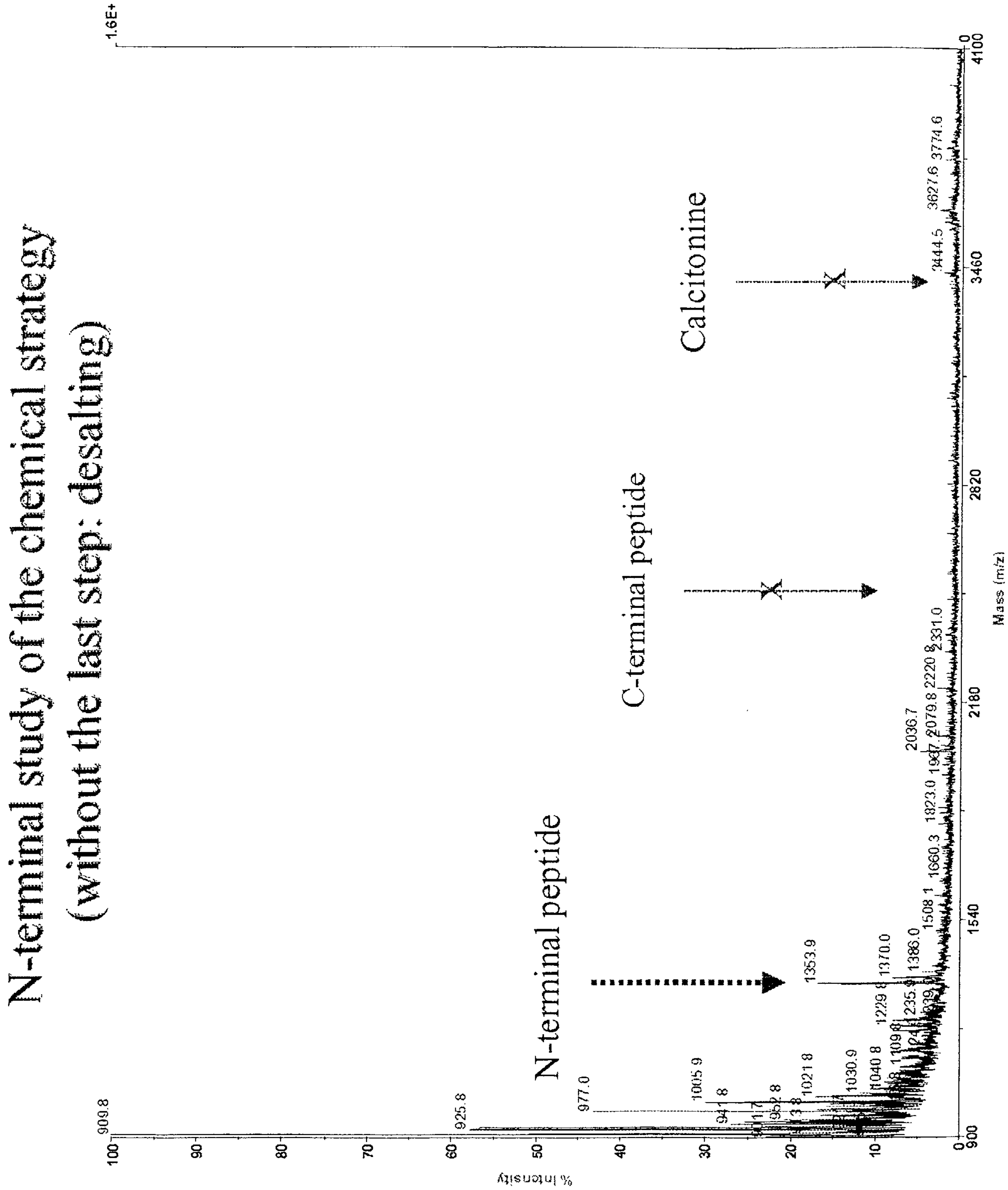
N-terminal study of the chemical strategy
(without the last step: desalting)
N-terminal peptide
C-terminal peptide
Calcitonine
% Intensity
Mass (m/z)

European Patent Office

# EUROPEAN SEARCH REPORT

Application Number
EP 01 30 4975

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X<br><br>Y | DE 43 44 425 A (SHIMADZU CORP) 30 June 1994 (1994-06-30)<br>* column 3, line 22 - column 5, line 16; claim 1; figure 1 * | 1-7,11, 23-25, 27,29,38<br>8-10,28, 30,31 | G01N33/68<br>C07K1/12 |
| X<br><br>Y | DATABASE WPI<br>Section Ch, Week 199534<br>Derwent Publications Ltd., London, GB;<br>Class B04, AN 1995-261288<br>XP002177419<br>& JP 07 165789 A (TORAY RES CENT KK), 27 June 1995 (1995-06-27)<br>* abstract * | 1-6,9, 11, 23-25, 27,29,38<br><br>8 | |
| X<br>Y | US 6 156 527 A (THOMPSON ANDREW HUGIN ET AL) 5 December 2000 (2000-12-05)<br>* the whole document * | 7,8<br>8,28,30, 31 | |
| Y | HOVING S ET AL: "A METHOD FOR THE CHEMICAL GENERATION OF N-TERMINAL PEPTIDE SEQUENCETAGS FOR RAPID PROTEIN IDENTIFICATION"<br>ANALYTICAL CHEMISTRY, AMERICAN CHEMICAL SOCIETY. COLUMBUS, US,<br>vol. 72, no. 5, 1 March 2000 (2000-03-01), pages 1006-1014, XP000958119<br>ISSN: 0003-2700<br>see abstract (-> multiple samples) and<br>* page 1008, paragraph 2 * | 9,10 | TECHNICAL FIELDS SEARCHED (Int.Cl.7)<br>G01N<br>C07K |
| A | US 5 141 474 A (FUJIMOTO SHINJI) 25 August 1992 (1992-08-25)<br>see Abstract | 12-22, 32-37, 39-48 | |

-/--

The present search report has been drawn up for all claims

| Place of search | Date of completion of the search | Examiner |
|---|---|---|
| MUNICH | 21 November 2001 | G. Willière |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 01 30 4975

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

# EUROPEAN SEARCH REPORT

Application Number
EP 01 30 4975

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | COHN J A ET AL: "CHEMICAL CHARACTERIZATION OF A PROTEIN-4-HYDROXY-2-NONENAL CROSS-LINK: IMMUNOCHEMICAL DETECTION IN MITOCHONDRIA EXPOSED TO OXIDATIVE STRESS" ARCHIVES OF BIOCHEMISTRY AND BIOPHYSICS, NEW YORK, US, US, vol. 328, no. 1, 1 April 1996 (1996-04-01), pages 158-164, XP001025844 ISSN: 0003-9861 see abstract and figure 1 ----- | 12-22, 32-37, 39-48 | |
| | | | TECHNICAL FIELDS SEARCHED (Int.Cl.7) |

The present search report has been drawn up for all claims

| Place of search | Date of completion of the search | Examiner |
|---|---|---|
| MUNICH | 21 November 2001 | G. Willière |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# LACK OF UNITY OF INVENTION SHEET B

Application Number
EP 01 30 4975

The Search Division considers that the present European patent application does not comply with the requirements of unity of invention and relates to several inventions or groups of inventions, namely:

1. Claims: 1-11, 23-31, 38

   Method or kit for characterising polypeptides comprisising the steps as mentionned in claims 1, 5, 38 and claims depending thereof.

2. Claims: 12-22, 32-37, 39-48

   Method or kit for characterising polypeptides comprisising a hindered Michael reagent as well as the use of a hindered Michael reagent for protecting epsilon amino groups in peptides and claims depending thereof.

## ANNEX TO THE EUROPEAN SEARCH REPORT ON EUROPEAN PATENT APPLICATION NO. EP 01 30 4975

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2001

| Patent document cited in search report | | Publication date | Patent family member(s) | | | Publication date |
|---|---|---|---|---|---|---|
| DE 4344425 | A | 30-06-1994 | JP | 6189784 | A | 12-07-1994 |
| | | | DE | 4344425 | A1 | 30-06-1994 |
| JP 7165789 | A | 27-06-1995 | NONE | | | |
| US 6156527 | A | 05-12-2000 | AU | 721390 | B2 | 29-06-2000 |
| | | | AU | 5674598 | A | 18-08-1998 |
| | | | CN | 1244218 | T | 09-02-2000 |
| | | | EP | 1027454 | A1 | 16-08-2000 |
| | | | WO | 9832876 | A1 | 30-07-1998 |
| | | | JP | 2001508872 | T | 03-07-2001 |
| US 5141474 | A | 25-08-1992 | JP | 1921809 | C | 07-04-1995 |
| | | | JP | 2026319 | A | 29-01-1990 |
| | | | JP | 6048017 | B | 22-06-1994 |
| | | | WO | 9000690 | A1 | 25-01-1990 |

EPO FORM P0459

For more details about this annex : see Official Journal of the European Patent Office, No. 12/82